(19) Europäisches Patentamt
European Patent Office
Office européen des brevets

(11) Veröffentlichungsnummer: **0 089 313**
**B1**

(12) # EUROPÄISCHE PATENTSCHRIFT

(45) Veröffentlichungstag der Patentschrift: **02.05.85**

(51) Int. Cl.[4]: **C 07 C 131/00**, A 01 N 35/10, C 07 D 317/18, C 07 D 319/06, C 07 D 321/02, A 01 N 43/20, A 01 N 43/32

(21) Anmeldenummer: **83810097.2**

(22) Anmeldetag: **09.03.83**

(54) **Neue Oximäther, Verfahren zu ihrer Herstellung, Mittel die die neuen Oximäther enthalten und ihre Verwendung.**

(30) Priorität: **15.03.82 CH 1609/82**

(43) Veröffentlichungstag der Anmeldung: **21.09.83 Patentblatt 83/38**

(45) Bekanntmachung des Hinweises auf die Patenterteilung: **02.05.85 Patentblatt 85/18**

(84) Benannte Vertragsstaaten: **AT BE CH DE FR GB IT LI NL**

(56) Entgegenhaltungen: **EP-A-0 011 047**

(73) Patentinhaber: **CIBA-GEIGY AG, Postfach, CH-4002 Basel (CH)**

(72) Erfinder: **Martin, Henry, Dr., Jupiterstrasse 17, CH-4123 Allschwil (CH)**
Erfinder: **Fricker, Urs, Baumgartenweg 8, CH-4460 Gelterkinden (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Oximäther, Verfahren zu ihrer Herstellung, Mittel zum Schutz von Kulturpflanzen vor der phytotoxischen Wirkung von Herbiziden, die die neuen Oximäther als aktive Komponente enthalten und ihre Verwendung.

Es ist bekannt, dass Herbizide aus den verschiedensten Stoffklassen, wie Triazine, Harnstoffderivate, Carbamate, Thiolcarbamate, Halogenacetanilide, Halogenphenoxyessigsäuren usw. bei der Anwendung in wirksamer Dosis häufig neben den zu bekämpfenden Unkräutern auch die Kulturpflanzen in gewissem Masse schädigen. Um diesem Problem zu begegnen, sind schon verschiedene Stoffe vorgeschlagen worden, welche befähigt sind, die schädigende Wirkung des Herbizids auf die Kulturpflanze spezifisch zu antagonisieren, d.h. die Kulturpflanze zu schützen, ohne dabei die Herbizidwirkung auf die zu bekämpfenden Unkräuter merklich zu beeinflussen. Dabei hat es sich gezeigt, dass die vorgeschlagenen Gegenmittel sowohl bezüglich der Kulturpflanzen als auch bezüglich des Herbizids und gegebenenfalls auch in Abhängigkeit von der Applikationsart oft sehr artspezifisch wirken, d.h. ein bestimmtes Gegenmittel eignet sich oft nur für eine bestimmte Kulturpflanze und einige wenige herbizide Stoffklassen.

So beschreibt die GB-PS Nr. 1277557 die Behandlung von Samen bzw. Sprösslingen von Weizen und Sorghum mit gewissen Oxamsäureestern und Amiden zum Schutz vor dem Angriff durch Alachlor (N-Methoxymethyl-N-chloracetyl-2,6-diäthylanilin). In den DE-OS Nrn. 1952910 und 2245471 sowie in der FR-PS Nr. 2021611 werden Gegenmittel zur Behandlung von Getreide-, Mais- und Reissamen zum Schutz gegen die schädigende Einwirkung von Herbizid wirksamen Thiolcarbamaten vorgeschlagen. Gemäss der DE-PS Nr. 1576676 und der US-PS Nr. 3131509 werden Hydroxyaminoacetanilide und Hydantoine für den Schutz von Getreidesamen gegenüber Carbamaten verwendet.

Die direkte pre- oder postemergente Behandlung gewisser Nutzpflanzen mit Gegenmitteln als Antagonisten bestimmter Herbizidklassen auf einer Anbaufläche ist in den DE-OS Nrn. 2141586 und 2218097 sowie im US-Patent Nr. 3867444 beschrieben.

Ferner können Maispflanzen gemäss der DE-OS Nr. 2402983 wirksam vor Schädigung durch Chloracetanilide geschützt werden, indem man dem Boden als Gegenmittel ein N-disubstituiertes Dichloracetamid zuführt.

Gemäss EP-A Nr. 11047 können auch Alkoximinobenzylcyanide, deren Alkoxygruppe u.a. durch eine acetalisierte Carbonylgruppe substituiert ist, als Mittel zum Schutz von Kulturpflanzen vor der schädigenden Wirkung von Herbiziden verschiedener Stoffklassen verwendet werden.

Gemäss vorliegender Erfindung werden neue Oximäther der Formel I

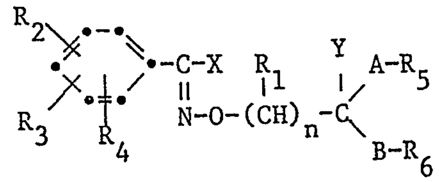
(I)

in welcher n 1 oder 2, $R_1$ und $R_2$ je Wasserstoff oder $C_1$-$C_4$-Alkyl, $R_3$ und $R_4$ je Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Alkylsulfonyl, $C_1$-$C_4$-Halogenalkylsulfinyl, $C_1$-$C_4$-Halogenalkylsulfonyl oder Nitro, $R_5$ und $R_6$ unabhängig voneinander je $C_1$-$C_4$-Alkyl, $C_1$-$C_4$-Halogenalkyl, $C_2$-$C_8$-Alkoxyalkyl, $C_3$-$C_4$-Alkenyl, $C_3$-$C_4$-Alkinyl oder zusammen eine 2- bis 6gliedrige Alkylen- oder Alkenylenbrücke, die 1- bis 4mal durch $C_1$-$C_4$-Alkyl, $C_1$-$C_4$-Halogenalkyl, $C_1$-$C_4$-Alkoxy, $C_2$-$C_4$-Alkenyloxy, $C_2$-$C_8$-Alkoxyalkyl oder $C_3$-$C_8$-Alkenyloxyalkyl substituiert sein kann, A und B unabhängig voneinander je Sauerstoff, Schwefel oder eines davon die Methingruppe, X einen fluorierten $C_1$-$C_3$-Alkylrest, der zusätzlich auch Chlor enthalten kann und Y Wasserstoff, $C_1$-$C_4$-Alkyl, $C_1$-$C_4$-Halogenalkyl, $C_1$-$C_4$-Alkoxy, $C_1$-$C_4$-Halogenalkoxy oder Phenyl.

Die als bzw. in den Resten $R_1$-$R_6$ vorkommenden $C_1$-$C_4$-Alkylgruppen können geradkettig oder verzweigt sein und bedeuten im einzelnen Methyl, Äthyl, n-Propyl, Isopropyl, n-Butyl, sek.-Butyl, Isobutyl und tert.-Butyl. Unter diesen Alkylgruppen sind $C_1$-$C_2$-Alkylgruppen bevorzugt und $R_1$ bedeutet als Alkylrest vorzugsweise Methyl.

Unter Halogen sind Fluor, Chlor, Brom und Jod, insbesondere aber Fluor und Chlor zu verstehen.

Der fluorierte Alkylrest X kann beispielsweise Difluormethyl, Trifluormethyl, Chlordifluormethyl, Tetrafluoräthyl, Pentafluoräthyl, Difluorchlorfluoräthyl und Heptafluorpropyl bedeuten. Unter den genannten Resten X sind perfluorierte Alkylreste bevorzugt, wobei ein Fluoratom durch Chloratom ersetzt sein kann. Besonders bevorzugte Reste X sind Trifluormethyl, Chlordifluormethyl und Pentafluoräthyl.

Je nach der Bedeutung von A, B und Y entstehen verschiedene Äther- oder Thioätherderivate.

Falls A und B je Sauerstoff, Y Wasserstoff und $R_5$ und $R_6$ je Alkyl-, Alkenyl- oder substituierte Alkylreste darstellen, erhält man Acetale; bilden $R_5$ und $R_6$ zusammen eine Alkylen- oder Alkenylenbrücke, so erhält man cyclische Acetale, wie 1,3-Dioxolane, 1,3-Dioxan, 1,3-Dioxepan beziehungsweise 1,3-Dioxep-5-on-derivate wie auch höhere Diox-Ringsysteme.

Sind A und B je Schwefel, Y Wasserstoff und $R_5$ und $R_6$ je ein Kohlenwasserstoffrest, erhält man Thioacetale; bilden 5 und 6 zusammen eine Kohlenwasserstoffbrücke, erhält man Dithioione und Diethiane.

Bedeuten A und B je Sauerstoff und Y Alkyl oder Phenyl, erhält man Ketale; wenn A und B Schwefel bedeuten, erhält man Thioketale.

Wenn A und B je Sauerstoff, Y Alkoxy und $R_5$

und $R_6$ je einen Kohlenwasserstoffrest bedeuten, erhält man einen Orthoester; bedeuten $R_5$ und $R_6$ zusammen eine Kohlenwasserstoffbrücke, erhält man cyclische Orthoester.

Es können A auch Sauerstoff, B Methylen, Y Wasserstoff und $R_5$ und $R_6$ zusammen eine Kohlenwasserstoffbrücke bedeuten, was zu Furan-, Dihydrofuran-, Tetrahydrofuran-, Pyran-, Di- oder Tetrahydropyran- und ähnlichen Sauerstoffringderivaten führt.

Für diese Verbindungstypen bestehen oft verschiedene Herstellungsverfahren.

Es sind folgende Varianten der Verbindungen der Formel I möglich

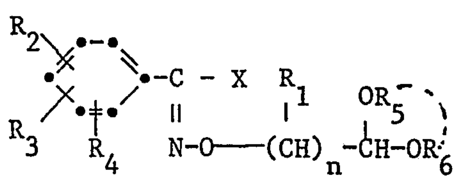

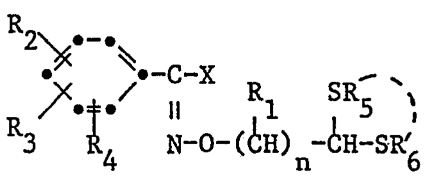

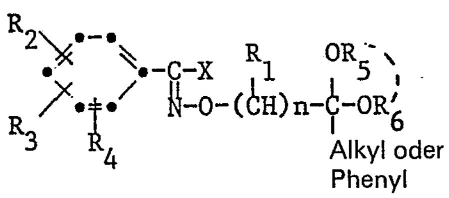

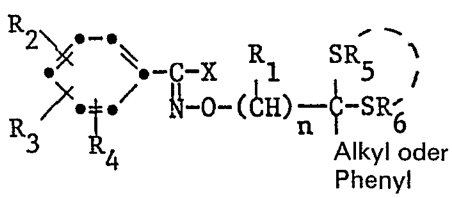

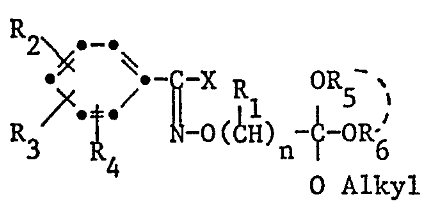

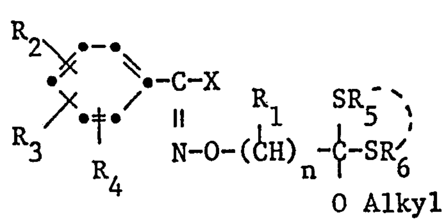

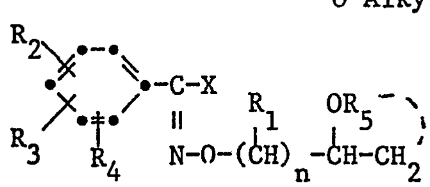

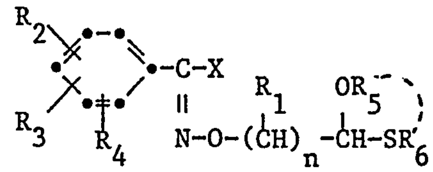

Die Oximäther der Formel I treten demnach in folgenden wichtigen Untergruppen in Erscheinung, beispielsweise als

a) Acetalderivate der Formel II

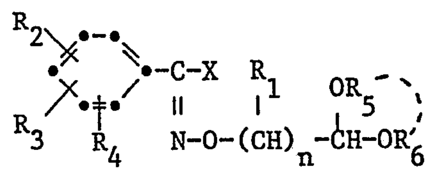

(II)

worin n, $R_1$, $R_2$, $R_3$, $R_4$ und X die unter Formel I gegebene Bedeutung haben und $R_5$ und $R_6$ unabhängig voneinander je $C_1$-$C_4$-Halogenalkyl, $C_2$-$C_8$-Alkoxyalkyl, $C_3$-$C_4$-Alkenyl, $C_3$-$C_4$-Alkinyl, d.h. beispielsweise gleich oder verschieden Methyl, Äthyl, Propyl, Isopropyl, Butyl, Chloräthyl, Bromäthyl, Methoxyäthyl, Allyl oder Propargyl bedeuten.

b) Orthoester der Formel III

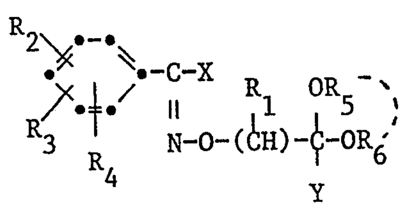

(III)

worin n, $R_1$, $R_2$, $R_3$, $R_4$ und X die unter Formel I gegebene Bedeutung haben, $R_5$ und $R_6$ unabhängig voneinander je $C_1$-$C_4$-Alkyl, $C_1$-$C_4$-Halogenalkyl, $C_2$-$C_8$-Alkoxyalkyl, $C_3$-$C_4$-Alkenyl oder $C_3$-$C_4$-Alkinyl und Y $C_1$-$C_4$-Alkoxy bedeuten.

c) Ringderivate, z.B. die 1,3-Dioxan-, 4,7-Dihydro-, 1,3-Dioxepinverbindungen der Formeln IV und IVa

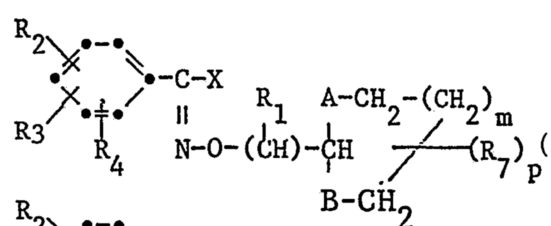

(IV)

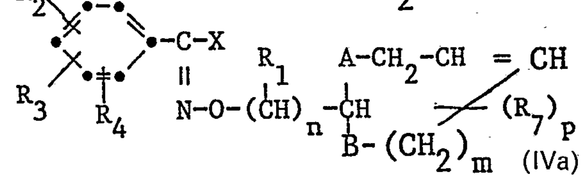

(IVa)

worin n, $R_1$, $R_2$, $R_3$, $R_4$, A, B und X die unter Formel I gegebene Bedeutung haben, m und p je 0 oder eine Zahl 1, 2 oder 3 bedeutet, $R_7$ $C_1$-$C_4$-Alkyl, $C_1$-$C_4$-Halogenalkyl, $C_2$-$C_8$-Alkoxyalkyl oder Alkenyloxyalkyl vorzugsweise Methyl bedeuten. Diejenigen Ringderivate sind bevorzugt, in denen A und B Sauerstoff bedeuten und die der Formel V entsprechen

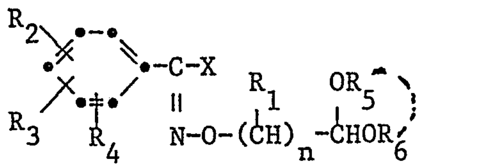

(V)

worin n, $R_1$, $R_2$, $R_3$, $R_4$ und X die unter Formel I gegebene Bedeutung haben und $R_5$ und $R_6$ zusammen eine 2- bis 6gliedrige Alkylen- oder Alkenylenbrücke bedeuten, die 1- bis 3mal durch $C_1$-$C_4$-Alkyl, $C_1$-$C_4$-Halogenalkyl, $C_2$-$C_8$-Alkoxyalkyl oder Alkenyloxyalkyl substituiert sein kann.

Unter diesen Verbindungen sind wiederum folgende bevorzugt:

d) Verbindungen, in welchen n für 1 steht, $R_1$, $R_2$, $R_4$ und Y je Wasserstoff darstellen, $R_3$ in 4-Stellung steht und Wasserstoff, Halogen, Methyl, Difluormethyl, Trifluormethyl, Chlordifluormethyl, Methoxy, Difluormethoxy, Trifluormethoxy, Chlordifluormethoxy, Tetrafluoräthyl, Tetrafluoräthoxy oder Trifluormethylsulfonyl bedeutet, A und B je Sauerstoff, X Trifluormethyl, Chlordifluormethyl oder Pentafluoräthyl darstellen und $R_5$ und $R_6$ die unter Formel I gegebene Bedeutung haben.

e) Verbindungen, in welche n für 1 steht, $R_1$, $R_2$, $R_4$ und Y je Wasserstoff darstellen, $R_3$ in 4-Stellung steht und Wasserstoff, Fluor, Chlor, Methyl, Difluormethyl, Trifluormethyl, Chlordifluormethyl, Methoxy, Difluormethoxy, Trifluormethoxy, Chlordifluormethoxy, Tetrafluoräthyl, Tetrafluoräthoxy oder Trifluormethylsulfonyl bedeutet, A und B je Sauerstoff, X Trifluormethyl, Chlordifluormethyl oder Pentafluoräthyl darstellen, während $R_5$ und $R_6$ zusammen eine gegebenenfalls 1- bis 4mal durch Methyl, Chlormethyl, $C_1$-$C_4$-Alkoxy oder $C_2$-$C_4$-Alkenyloxy substituierte 1,2-Äthylen- oder 1,3-Propylengruppe bedeuten.

f) Verbindungen, in welchen n für 1 steht, $R_1$, $R_2$ und $R_4$ Wasserstoff darstellen, $R_3$ in 4-Stellung steht und Wasserstoff, Fluor, Chlor, Methyl, Trifluormethyl oder Trifluormethoxy bedeutet, A und B je Sauerstoff, X Trifluormethyl, Chlordifluormethyl oder Pentafluoräthyl ist und $R_5$ und $R_6$ zusammen eine gegebenenfalls durch 1 bis 4 Methylengruppen substituierte 1,2-Äthylengruppe darstellen.

g) Verbindungen, in welchen n für 1 steht, $R_1$, $R_2$, $R_4$ und Y je Wasserstoff darstellen, $R_3$ in 2-Stellung steht und Fluor oder Chlor bedeutet, A und B je Sauerstoff und $R_5$ und $R_6$ zusammen eine gegebenenfalls durch 1 bis 4 Methylgruppen substituierte 1,2-Äthylengruppe oder 1,3-Propylengruppe bedeuten, und

h) Verbindungen, in welchen n für 1 steht, $R_1$, $R_2$, $R_4$ und Y je Wasserstoff darstellen, $R_3$ in 3-Stellung steht und Trifluormethyl oder Nitro bedeutet, A und B je Sauerstoff, X Trifluormethyl, Chlordifluormethyl oder Pentafluoräthyl ist und $R_5$ und $R_6$ zusammen eine gegebenenfalls durch 1 bis 4 Methylgruppen substituierte 1,2-Äthylengruppe oder 1,3-Propylengruppe bedeuten.

Bevorzugte Einzelverbindungen sind:

1-Phenyl-1-(1,3-dioxolan-2-ylmethoximino)-2,2,2-trifluoräthan

1-(4-Chlorphenyl)-1-(1,3-dioxolan-2-ylmethoximino)-2,2,2-trifluoräthan

1-(4-Chlorphenyl)-1-(2,2-diäthoxyäthoximino)-2,2,2-trifluoräthan

1-(4-Chlorphenyl)-1-(2,2-dimethoxyäthoxyäthoximino)-2,2,2-trifluoräthan

1-(4-Fluorphenyl)-1-(1,3-dioxolan-2-ylmethoximino)-2,2,2-trifluoräthan

1-(3,4-Dimethylphenyl)-1-(1,3-dioxolan-2-ylmethoximino)-2,2,2-trifluoräthan

1-(4-Fluorphenyl)-1-(1,3-dioxolan-4-methyl-2-ylmethoximino)-2,2,2-trifluoräthan

1-Phenyl-1-(1,3-dioxan-2-ylmethoximino)-2,2,2-trifluoräthan

1-(3-Trifluormethylnaphthyl)-1-(1,3-dioxan-2-ylmethoximino)-2,2,2-trifluoräthan

1-Phenyl-1-(1,3-dioxolan-2-ylmethoximino)-2,2,3,3,3-pentafluorpropan

1-(4-Chlorphenyl)-1-(1,3-dioxolan-2-yläthoximino)-2,2,2-trifluoräthan

1-(4-Fluorphenyl)-1-(1,3-dioxan-2-ylmethoximino)-2,2,2-trifluoräthan

1-(4-Fluorphenyl)-1-(1,3-dioxan-2-ylmethoximino)-2,2,2-trifluoräthan

1-(4-Fluorphenyl)-1-(1,3-dioxolan-2-yläthoximino)-2,2,2-trifluoräthan

1-(4-Chlorphenyl)-1-(1,3-dioxolan-4,5-dimethyl-2-ylmethoximino)-2,2,2-trifluoräthan

1-(3-Trifluormethylphenyl)-1-(1,3-dioxolan-4-äthyl-2-ylmethoxyimino)-2,2,2-trifluoräthan

Die neuen Oximäther der Formel I werden erfindungsgemäss hergestellt, indem man ein Salz eines Oxims der Formel VI

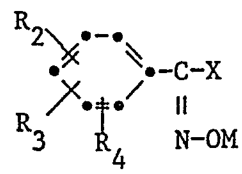

(VI)

in welcher M ein Alkalimetall- oder Erdalkalimetallkation darstellt und $R_2$, $R_3$, $R_4$ und X die oben angegebene Bedeutung haben, mit einem Halogenalkyläther der Formel VII

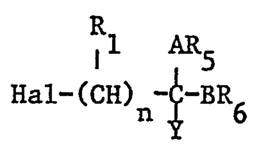

(VII)

in welcher Hal ein Halogenatom, insbesondere ein Chloratom oder ein Bromatom, bedeutet und A, B, n, $R_1$, $R_5$ und Y die oben angegebene Bedeutung haben, umsetzt. Als Salze eines Oxims der Formel VI sind insbesondere die Natrium- und Kaliumsalze geeignet. Die Umsetzung des Oxims der Formel VI mit dem Halogenalkyläther der Formel VII wird vorteilhaft in einem inerten organischen Lösungsmittel durchgeführt. Besonders geeignet sind polare Lösungsmittel, wie Acetonitril, Dimethylformamid und Dimethylsulfoxid. Die Reaktanten werden in der Regel in äquimolarer Menge eingesetzt. Es kann jedoch auch zum vollständigen Ablauf der Reaktion der eine oder der andere

Reaktionspartner im Überschuss eingesetzt werden. Die Umsetzung wird vorteilhaft bei erhöhter Temperatur, vorzugsweise bei 60 bis 70° C durchgeführt.

Die Oxime der Formel VI können in bekannter Weise durch Umsetzung der entsprechenden Ketone mit Hydroxylamin hergestellt werden. Die hierfür benötigten Ketone können ihrerseits durch Umsetzung einer Grignard-Verbindung der Formel VIII

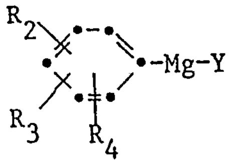 (VIII)

in welcher Y Chlor, Brom oder Jod bedeutet und $R_2$, $R_3$ und $R_4$ die oben angegebene Bedeutung haben, mit einer vom Rest X gemäss obiger Definition abgeleiteten Carbonsäure X-COOH, einem Säurechlorid X-COCl oder einem Nitril X-CN erhalten werden (vgl. US-Patent Nr. 3748361). Ferner ist es möglich, zur Herstellung der Oxime der Formel VI geeignete Ketone durch Umsetzung eines Benzols der Formel IX

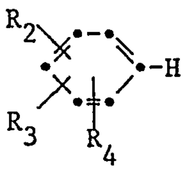 (IX)

in welcher $R_2$, $R_3$ und $R_4$ die oben angegebene Bedeutung haben, mit einem vom Rest X gemäss obiger Definition abgeleiteten Carbonsäurechlorid X-COCl in Gegenwart von Aluminiumchlorid zu gewinnen.

Oxime der Formel VI, die zur Herstellung der neuen Oximäther der Formel I geeignet sind, sind beispielsweise:

1-Phenyl-1-hydroximino-2,2,2-trifluoräthan

1-(4-Methylphenyl)-1-hydroximino-2,2,2-trifluoräthan

1-(4-Chlorphenyl)-1-hydroximino-2,2,2-trifluoräthan

1-(4-Fluorphenyl)-1-hydroximino-2,2,2-trifluoräthan

1-(4-Trifluormethylphenyl)-1-hydroximino-2,2,2-trifluoräthan

1-(3-Trifluormethylphenyl)-1-hydroximino-2,2,2-trifluoräthan

1-(4-Methoxyphenyl)-1-hydroximino-2,2,2-trifluoräthan

1-(4-Trifluormethoxyphenyl)-1-hydroximino-2,2,2-trifluoräthan

1-(3-Nitrophenyl)-1-hydroximino-2,2,2-trifluoräthan

1-(3,4-Dimethylphenyl)-1-hydroximino-2,2,2-trifluoräthan

1-(3,4-Dichlorphenyl)-1-hydroximino-2,2,2-trifluoräthan

1-(4-Chlorphenyl)-1-hydroximino-2,2-difluoräthan

1-(4-Chlorphenyl)-1-hydroximino-2-chlor-2,2-difluoräthan

1-(4-Methoxyphenyl)-1-hydroximino-2-chlor-2,2-difluoräthan

1-(4-Trifluormethoxyphenyl)-1-hydroximino-2-chlor-2,2-difluoräthan

1-(3-Nitrophenyl)-1-hydroximino-2-chlor-2,2-difluoräthan

1-Phenyl-1-hydroximino-2,2,3,3,3-pentafluorpropan

1-(4-Methylphenyl)-1-hydroximino-2,2,3,3,3-pentafluorpropan

1-(4-Chlorphenyl)-1-hydroximino-2,2,3,3,3-pentafluorpropan

1-(3-Nitrophenyl)-1-hydroximino-2,2,3,3,3-pentafluorpropan

1-Phenyl-1-hydroximino-2,2,3,3,4,4,4-heptafluorbutan

1-(4-Chlorphenyl)-1-hydroximino-2,2,3,3,4,4,4-heptafluorbutan

1-(2-Chlorphenyl)-1-hydroximino-2,2,2-trifluoräthan

1-(2-Fluorphenyl)-1-hydroximino-2,2,2-trifluoräthan

Die Halogenalkyläther der Formel VII können hergestellt werden, indem man ein halogeniertes Alkanol der Formel VIIa

$$\mathrm{Hal-}\underset{\textstyle |}{\overset{R_1}{}}\!\!\!\!\mathrm{(CH)_n-CHO} \qquad \text{(VIIa)}$$

in welcher n, Hal und $R_1$ die oben angegebene Bedeutung haben, in bekannter Weise mit einem $C_1$-$C_4$ Alkanol oder Mercaptan oder einem entsprechenden 1,2-, 1,3-Alkandiol, 1,4-Alkandiol, 1,5-Alkandiol, 1,4-Alkenyldiol oder einem entsprechenden Dimercaptan umsetzt.

Geeignete Halogenacetale der Formel III sind beispielsweise:

1,1-Dimethoxy-2-bromäthan

1,1-Dimethoxy-2-brompropan

1,1-Diäthoxy-2-bromäthan

1,1-Dimethoxy-3-brompropan

1,1-Diäthoxy-3-brombutan

1,3-Dioxolan-2-ylmethylbromid

1-(1,3-Dioxolan-2-yl)-1-bromäthan

1-(1,3-Dioxolan-2-yl)-2-bromäthan

1-(1,3-Dioxolan-2-yl)-2-brompropan

4,5-Dimethyl-1,3-dioxolan-2-ylmethylbromid

4,4,5,5-Tetramethyl-1,3-dioxolan-2-ylmethylbromid

4,4-Dimethyl-1,3-dioxolan-2-ylmethylbromid

1-(4,5-Dimethyl-1,3-dioxolan-2-yl)-1-bromäthan

1-(4,5-Dimethyl-1,3-dioxolan-2-yl)-2-bromäthan

1-(4,5-Dimethyl-1,3-dioxolan-2-yl)-2-brompropan

1,3-Dioxan-2-ylmethylbromid

1-(1,3-Dioxan-2-yl)-1-bromäthan

1-(1,3-Dioxan-2-yl)-2-bromäthan

1-(1,3-Dioxan-2-yl)-2-brompropan

5,5-Dimethyl-1,3-dioxan-2-ylmethylbromid

4,4-Dimethyl-1,3-dioxan-2-ylmethylbromid

4,4,6-Trimethyl-1,3-dioxan-2-ylmethylbromid

1-(4,4,6-Trimethyl-1,3-dioxan-2-yl)-1-bromäthan

1-(5,5-Dimethyl-1,3-dioxan-2-yl)-1-brom-äthan
1-(5,5-Dimethyl-1,3-dioxan-2-yl)-2-brom-äthan
1-(5,5-Dimethyl-1,3-dioxan-2-yl)-2-brompropan.

Die obgenannten reaktionsfähigen Acetale stellen eine nicht limitierende Auswahl dar. Man kann sie z.B. herstellen durch Umsetzen von Bromacetaldehyddimethyl- oder- diäthylacetalen mit 1,2-, 1,3-, 1,4- oder 1,5-Alkan- oder Alkendiolen, welche gegebenenfalls durch Methyl, Chlormethyl, $C_1$-$C_4$-Alkoxymethyl, $C_2$-$C_4$-Alkenyloxymethyl oder Nitro substituiert sein können.

Als Diole, Dimerkaptane oder gemischte Merkaptoalkohole kommen beispielsweise folgende in Frage:

1,2-Propandiol,
(+) S-1,2-Propandiol
(−) S-1,2-Propandiol
3-Fluor-1,2-propandiol
3-Chlor-1,2-propandiol
3-Brom-1,2-propandiol
3-Iod-1,2-propandiol
3-Methoxy-1,3-propandiol
3-Äthoxy-1,3-propandiol
3-Isopropoxy-1,3-propandiol
2-Nitro-1,3-propandiol
(+) R-1,2-Butandiol
(−) S-1,2-Butandiol
1,2-Butandiol
1,3-Butandiol
1,4-Butandiol
meso-2,3-Butandiol
(−) 2S, 3S 2,3-Butandiol
1-Buten-2,3-diol
2,3-Pentandiol
2-Methyl-1,2-butandiol
2-Methyl-2,3-butandiol
3-Allyloxy-1,2-propandiol
3-Methallyloxy-1,2-propandiol
3-Propargyloxy-1,2-propandiol
3-Acetoxy-1,3-propandiol
3-Methylmercapto-1,2-propandiol
3-Chlorallylmercapto-1,2-propandiolglycerol
1,3-Propandiol
2-Chlor-1,3-propandiol
2-Brom-1,3-propandiol
2-Buten-1,4-diol
2-Hydroxymethyl-2-propen-1-ol
2-Hydroxymethyl-2-buten-1-ol
2-Methyl-1,2-propandiol
3-Chlor-2-methyl-1,2-propandiol
3-Chlor-2-chlormethyl-1,2-propandiol
3-Methyl-1,3-propandiol
2-Methyl-2-nitro-1,3-propandiol
1,2-Pentandiol
2,4-Pentandiol
2-Methyl-1,3-butandiol
2-Methyl-2,4-butandiol
2-Methyl-3,4-butandiol
1,4-Dichlor-2-methyl-2,3-butandiol
4-Iod-2-methyl-2,3-butandiol
2,2-Dimethyl-1,3-propandiol
2,4-Hexandiol
3-Methyl-2,4-pentandiol
1,5-Hexan-3,4-diol
2-Propanol-1-thiol
3-Propanol-1-thiol
1,3-Propandithiol
2,3-Butandithiol
1-Methyl-1,2-propandithiol
1-Buten-2,3-dithiol
2-Methyl-1,2-butandithiol
2-Äthyl-1,3-propandiol
4-Brom-2-methyl-2,3-butandiol
2,2-Dimethyl-1,3-propandiol
2,2-Bischlormethyl-1,3-propandiol
2-Methyl-2,3-pentandiol
2,2-Dimethyl-3,4-butandiol
2-Äthanol-1-thiol
3-Chlor-2-propanol-1-thiol
1,2-Äthandithiol
1,2-Butandithiol
1,4-Butendithiol
2-Buten-1,4-dithiol
2,3-Pentandithiol
2-Methyl-2,3-butandithiol

Verbindungen der Formel I können auch in andere umgewandelt werden. Eine 4,7-Dihydro-1,3-dioxepinverbindung der Formel I wird erfindungsgemäss beispielsweise nach folgendem Schema hergestellt

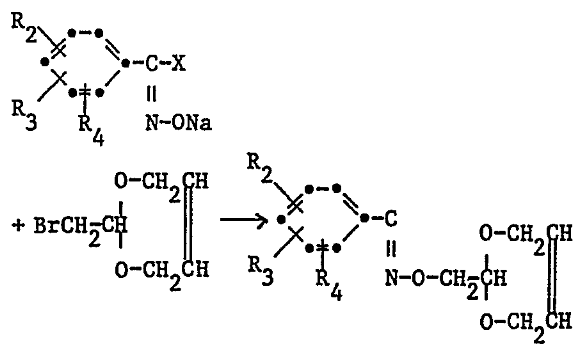

Durch Behandlung dieser Verbindung bei 100° C mit einem Rutheniumkatalysator kann gemäss einem in „Tetrahydron", *21* (1980), 4922, beschriebenen Verfahren die Doppelbindung verschoben werden, so dass die entsprechende 4,5-Dihydro-1,3-dioxepinverbindung entsteht.

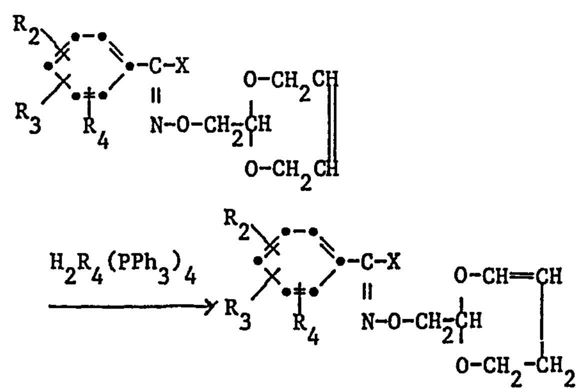


Zur Herstellung der Verbindungen der Formel I, in denen Y einen Alkyl- oder Phenylrest bedeutet, sezt man erfindungsgemäss das Oximsalz der Formel VI mit einem Halogenalkyl oder -phenylketal

oder -thioketal vorzugsweise einem Brom- oder Idoketal um, nach dem Schema

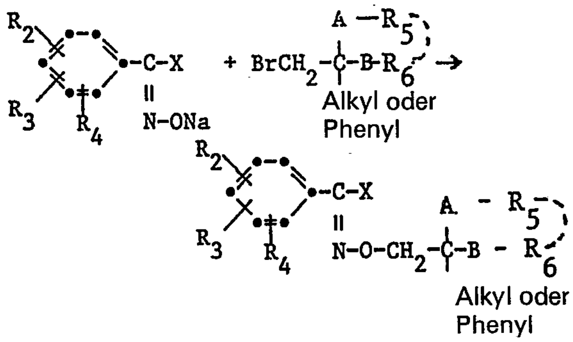


Solche Halogenketale sind aus der Literatur bekannt. Das α-Bromacetonketal kann gemäss der in „Synthesis", *1982*, 309, beschriebenen Methode hergestellt werden, andere Ketale, z.B. 1-Brompinakolinäthylenketal oder 1-Bromacetophenonäthylenketal können nach der Methode von „J. prakt. Chemie", *156* (1940), 121, erhalten werden.

Diese Verbindungen können ebenfalls durch Kondensation von Oximen z.B. mit Halogenketonen wie Bromaceton und nachträglicher Ketalisierung gewonnen werden. Eine weitere Methode zur Herstellung solcher Verbindungen ist beispielsweise die Umsetzung mittels 2-Methoxy-1,3-dioxolan analog zu „J. of med. Chem.", 25 (1982), 12, gemäss dem Schema

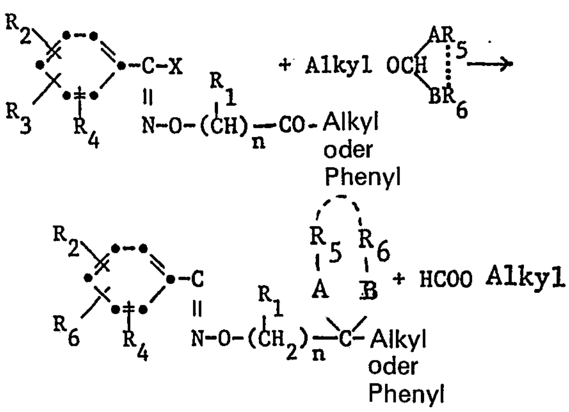


Die Orthoester der Formel I (Verbindungen, in denen Y Alkoxy bedeutet) werden erfindungsgemäss hergestellt durch Umsetzung des Oximsalzes der Formel VI mit einem Halogenorthocarbonsäureester, beispielsweise der Monobromorthoessigsäuretriäthylester analog „J. Am. Chem. Soc.", *59* (1937), 1273, und dem Schema

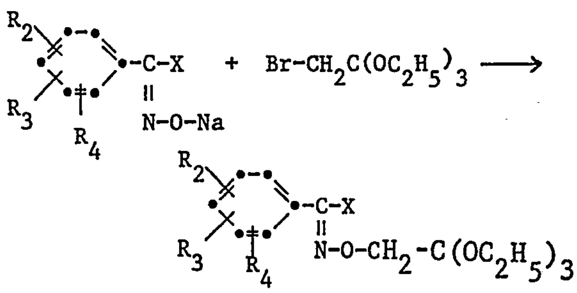

Durch Umsetzen der Halogenorthocarbonsäureester mit geeigneten Diolen und Mercaptanen werden gemischte Ester erhalten wie beispielsweise der Bromorthoessigsäureglykoläthylester oder der Bromorthoessigsäuredimethylthioäthylester welche zur Umsetzung mit dem Oximsalz der Formel VI geeignet sind. Orthoester der Formel I kann man beispielsweise gemäss einer im „J. Am. Chem. Soc.", *77* (1955), 4574, beschriebenen Methode auch durch Umsetzen eines Cyanoalkyloxims zum Iminoesterhydrochlorid und anschliessender Kondensation mit einem Diol wie z.B. Glykol nach dem Schema

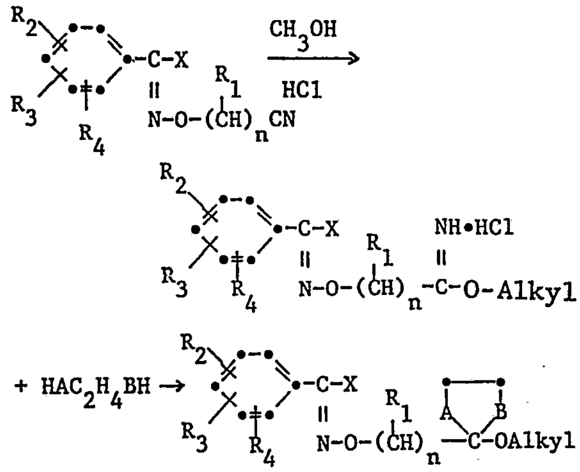


Die Herstellung von Verbindungen der Formel I, in denen A und/oder B Schwefel bedeuten, geschieht nach ähnlichen Arbeitsmethoden wie diejenige der Acetalverbindungen. Durch Verwendung von Thiolen oder Dithiolen kann man einfache Thioacetale, Thioketale, Dithiolane und Dithiane herstellen. Besonders jedoch eignet sich die Umesterung aliphatischer Dialkylacetale wie beispielsweise mit einem Mercaptan z.B. eines Oximdimethoxyäthylesters mit einem Mercaptan wie 1,2-Äthylendithiol im folgenden Schema

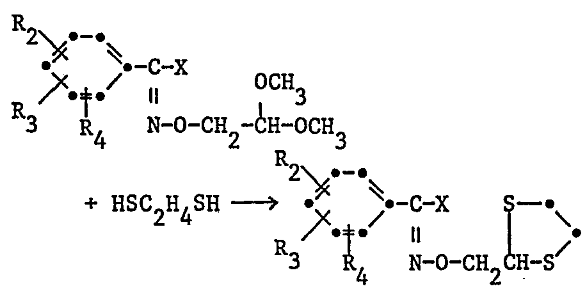

Man kann dieselben cyclischen Thioacetalverbindungen ebenfalls durch Umsetzen der entsprechenden Acetale mit Dithiolen erhalten, nach dem Schema

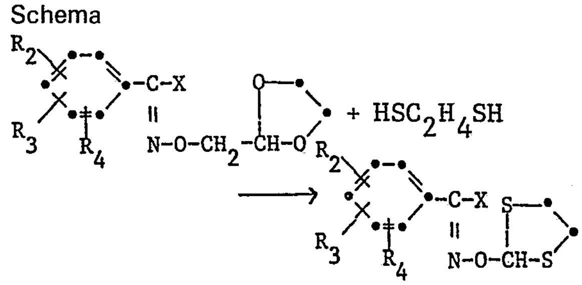

Verbindungen der Formel I, in denen A Sauerstoff, B Methylen und Y Wasserstoff und $R_5$ und $R_6$ zusammen ein Kohlenwasserstoff-Brückenglied bilden, können durch Umsetzen der Oximsalze der Formel VI mit entsprechenden 2-α-Halogenalkyl-sauerstoffringen wie z.B. 2-Brommethylfuran, 2-Brommethylpyran, 2-Bromethyloxepan gemäss dem Schema

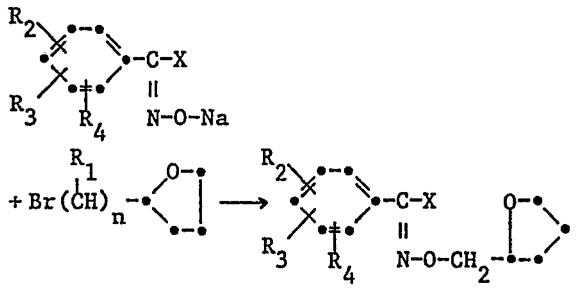

Anstatt wie in den vorhergehenden Verfahren von Oximsalzen der Formel VI auszugehen, kann man auch von den diesen Oximen zugrunde liegenden Ketonen ausgehen und diese mit O-substituierten Hydroxylaminen umsetzen, gemäss dem Schema

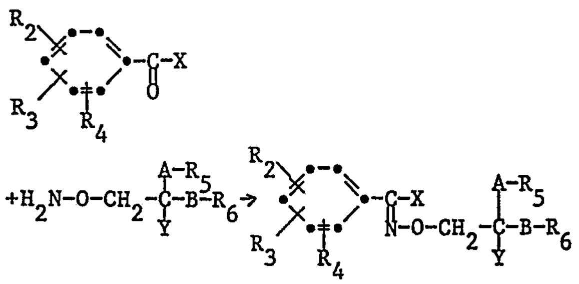

Ferner kann man solche Phenone mit entsprechend substituierten Acetonoximäthern umacetalisieren, um zu Verbindungen der Formel I zu kommen

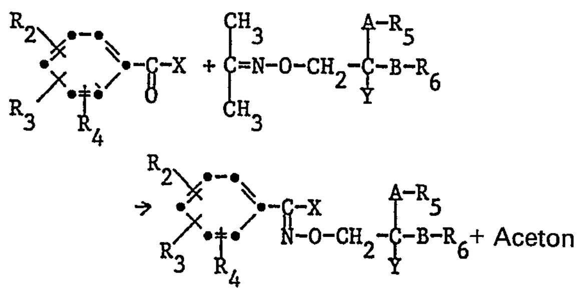


Die neuen Oximäther der Formel I besitzen in hervorragendem Masse die Eigenschaft, Kulturpflanzen vor Schädigung durch Agrarchemikalien zu schützen. Dieser Schutz erstreckt sich insbesondere auf Herbizide der verschiedenen Stoffklassen, darunter 1,3,5-Triazine, 1,2,4-Triazinone, Phenylharnstoffderivate, Carbamate, Thiolcarbamate, Phenoxyessigsäureester, Phenoxypropionsäureester, Halogenacetanilide, Halogenphenoxyessigsäureester, substituierte Phenoxyphenoxyessigsäureester und -propionsäureester, substituierte Pyridyloxyphenoxyessigsäureester und -propionsäureester, Benzoesäurederivate usw., sofern diese nicht oder ungenügend kulturtolerant sind. Die neuen Oximäther der Formel I sind vor allem zum Schützen von Kulturpflanzen gegen die schädigende Wirkung von Halogenacetaniliden und Thiolcarbamaten geeignet. Die Oximäther der Formel I können daher in bezug auf ihre Anwendung in Kombination mit den vorgenannten Herbiziden als Gegenmittel oder Antidotes oder auch als Safener bezeichnet werden.

Von den Verbindungen der Formel I existieren verschiedene isomere Formen. Als Oximderivate liegen diese Verbindungen in der Syn- bzw. Antiform, bzw. als deren Gemische vor (E- und Z-Form s. R. S. Cohn *et al.*, „Ang. Chemie Int. Ed.", *5* [1966], 385, oder „Experientia", *12* [1956], S. 81).

Je nach der Substitution der Verbindungen der Formel I besteht ein asymmetrisches C-Atom und es können zwei enatiomere Formen vorliegen. Im allgemeinen entsteht bei der Herstellung ein Gemisch beider Enantiomeren, welches sich auf übliche Weise in die optischen Antipoden auftrennen lässt.

Die monosubstituierten Dioxolane und Dioxane führen zu weiteren cis-trans-Isomeren beziehungsweise zu deren Gemischen. Zweifachsubstituierte Dioxolane führen zu cis-Syn und zu cis-Anti sowie trans-Isomeren bzw. deren Gemischen. Durch die Auswahl der Ausgangsmaterialien, beispielsweise durch Verwendung reiner Z-Form der Oxime lassen sich die Isomeren vermindern. Diese verschiedenen Isomeren sowie deren Gemische sind auch Gegenstand der Erfindung.

Ein solches Gegenmittel oder Andidote der Formel I kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation der phytotoxischen Chemikalie erfolgen. Die Behandlung der Pflanze kann jedoch auch durch gleichzeitige Applikation von phytotoxischer Chemikalie und Gegenmittel (Tankmischung) erfolgen. Die preemergente Behandlung schliesst sowohl die Behandlung der Anbaufläche vor der Aussaat (ppi = *pre plant incorporation*) als auch die Behandlung der angesäten, aber noch nicht bewachsenen Anbauflächen ein.

Die Aufwandmengen des Gegenmittels im Verhältnis zum Herbizid richten sich weitgehend nach der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1:100 bis 5:1. In der Regel wird bei einem Mengenverhältnis von Gegenmittel zu Herbizid von 1:1 bis 1:20 die volle Schutzwirkung erreicht. Bei der Samenbeizung und ähnlichen gezielten Schutzmassnahmen werden jedoch weit geringere Mengen Gegenmittel im Vergleich mit den später pro Hektar Anbaufläche verwendeten Mengen an Herbizid

benötigt. Im allgemeinen werden bei der Samenbeizung pro Kilo Samen 0,1-10 g Gegenmittel benötigt. In der Regel wird mit 0,1-2 g Gegenmittel pro Kilo Samen bereits die volle Schutzwirkung erreicht. Falls das Gegenmittel kurz vor der Aussaat durch Samenquellung appliziert werden soll, so werden zweckmässig Lösungen des Gegenmittels, welche den Wirkstoff in einer Konzentration von 1-10 000 ppm enthalten, verwendet.

In der Regel wird mit Konzentrationen des Gegenmittels von 100-1000 ppm die volle Schutzwirkung erreicht.

In der Regel liegt zwischen protektiven Massnahmen, wie Samenbeizung und Behandlung von Stecklingen mit einem Gegenmittel der Formel I, und der möglichen späteren Feldbehandlung mit Agrarchemikalien ein längerer Zeitraum. Vorbehandeltes Saat- und Pflanzengut kann später in Landwirt, Gartenbau und Forstwirtschaft mit unterschiedlichen Chemikalien in Berührung kommen. Die Erfindung bezieht sich daher auch auf protektive Mittel für Kulturpflanzen, die als Wirkstoff ein Gegenmittel der Formel I zusammen mit üblichen Trägerstoffen enthalten. Solche Mittel können gegebenenfalls zusätzlich jene Agrarchemikalien enthalten, vor deren Einfluss die Kulturpflanze geschützt werden soll.

Als Kulturpflanzen gelten im Rahmen vorliegender Erfindung alle Pflanzen, die in irgendeiner Form Ertragsstoffe, wie Samen, Wurzeln, Stengel, Knollen, Blätter, Blüten, ferner Inhaltsstoffe, wie Öle, Zucker, Stärke, Eiweiss usw., produzieren und zu diesem Zweck angebaut werden. Zu diesen Pflanzen gehören beispielsweise sämtliche Getreidearten, wie Weizen, Roggen, Gerste und Hafer, daneben vor allem Reis, Kulturhirse, Mais, Baumwolle, Zuckerrüben, Zuckerrohr, Soja, Bohnen und Erbsen.

Das Gegenmittel kann überall dort eingesetzt werden, wo eine Kulturpflanze der vorgenannten Art vor der schädlichen Wirkung einer Agrarchemikalie geschützt werden soll. Dabei kommen als Agrarchemikalien, wie bereits ausgeführt, in erster Linie Herbizide der verschiedensten Stoffklassen, insbesondere jedoch Halogenacetanilide und Thiolcarbamate in Betracht.

Halogenacetanilide, deren schädigende Wirkung gegenüber Kulturpflanzen mit Hilfe der neuen Oximäther der Formel I aufgehoben werden kann, sind bereits in grosser Zahl bekannt geworden (vgl. z.B. DE-A Nrn. 2305495, 2328340, 2212268, 2726253 und 2805757 sowie US-PS Nrn. 3946044, 4022608 und 4039314). Solche Halogenacetanilide können durch die folgende allgemeine Formel XIV beschrieben werden

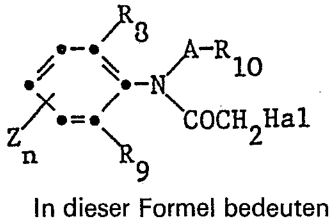

(XIV)

In dieser Formel bedeuten Hal Halogen, insbesondere Chlor oder Brom, $R_8$ und $R_9$ unabhängig voneinander je Wasserstoff, Halogen, niederes Alkyl, Alkoxy, Alkylthio, Halogenalkyl, Alkoxyalkyl oder Alkylthioalkyl, Z Wasserstoff, Halogen, niederes Alkyl, Alkoxy, Alkylthio, Halogenalkyl, Alkoxyalkyl oder Alkylthioalkyl, wobei die vorgenannten Reste Z vorzugsweise in 3-Stellung in bezug auf das Stickstoffatom stehen, n 0 bis 3, A Alkylen, insbesondere Methylen, 1,1- und 1,2-Äthylen, wobei 1,2-Äthylen durch 1 bis 2 niedere Alkylgruppen substituiert sein kann, und $R_{10}$ niederes Alkoxy, Hydroxycarbonyl, Alkoxycarbonyl, Carbamoyl, N-Alkylcarbamoyl, N,N-Dialkylcarbamoyl, Cyano, ein gegebenenfalls substituierter stickstoffhaltiger heterocyclischer Rest, Alkanoyl, gegebenenfalls substituiertes Benzoyl, gegebenenfalls substituiertes 1,3,4-Oxadiazol-2-yl, 1,3,4-Thiadiazol-2-yl, 1,3,4-Triazol-3-yl oder 1,3,4-Triazol-1-yl bedeutet.

Als einzelne Vertreter solcher Halogenacetanilide seien beispielsweise die folgenden genannt:

N-Äthoxymethyl-N-chloracetyl-2-äthyl-6-methylanilin

N-Chloracetyl-N-methoxymethyl-2,6-diäthylanilin

N-Chloracetyl-N-(2-methoxyäthyl)-2,6-dimethylanilin

N-(2-Allyloxyäthyl)-N-chloracetyl-2,6-dimethylanilin

N-Chloracetyl-N-(2-n-propoxyäthyl)-2,6-dimethylanilin

N-Chloracetyl-N-(2-isopropoxyäthyl)-2,6-dimethylanilin

N-Chloracetyl-N-(2-methoxyäthyl)-2-äthyl-6-methylanilin

N-Chloracetyl-N-(methoxyäthyl)-2,6-diäthylanilin

N-(2-Äthoxyäthyl)-N-chloracetyl-2-äthyl-6-methylanilin

N-Choracetyl-N-(2-methoxy-1-methyläthyl)-2-methylanilin

N-Chloracetyl-N-(2-methoxy-1-methyläthyl)-2,6-dimethylanilin

N-Chloracetyl-N-(2-methoxy-1-methyläthyl)-2,6-diäthylanilin

N-Chloracetyl-N-(2-methoxy-1-methyläthyl)-2-äthyl-6-methylanilin

N-(2-Äthoxyäthyl)-N-chloracetyl-2,6-diäthylanilin

N-Chloracetyl-N-(2-n-propoxyäthyl)-2-äthyl-6-methylanilin

N-Chloracetyl-N-(2-n-propoxyäthyl)-2,6-diäthylanilin

N-Chloracetyl-N-(2-isopropoxyäthyl)-2-äthyl-6-methylanilin

N-Äthoxycarbonylmethyl-N-chloracetyl-2,6-dimethylanilin

N-Äthoxycarbonylmethyl-N-chloracetyl-2,6-diäthylanilin

N-Chloracetyl-N-(2-methoxy-1-methyläthyl)-2,6-diäthylanilin

N-Chloracetyl-N-(2-methoxy-1-methyläthyl)-2-äthyl-6-methylanilin

N-(2-Äthoxyäthyl)-N-chloracetyl-2,6-diäthylanilin

N-Chloracetyl-N-(2-n-propoxyäthyl)-2-äthyl-6-methylanilin

N-Chloracetyl-N-(2-n-propoxyäthyl)-2,6-diäthylanilin
N-Chloracetyl-N-(2-isopropoxyäthyl)-2-äthyl-6-methylanilin
N-Äthoxycarbonylmethyl-N-chloracetyl-2,6-dimethylanilin
N-Äthoxycarbonylmethyl-N-chloracetyl-2,6-diäthylanilin
N-Chloracetyl-N-methoxycarbonylmethyl-2,6-dimethylanilin
N-Chloracetyl-N-(2,2-diäthoxyäthyl)-2,6-dimethylanilin
N-Chloracetyl-N-(2-methoxy-1-methyläthyl)-2,3-dimethylanilin
N-(2-Äthoxyäthyl)-N-chloracetyl-2-methylanilin
N-Chloracetyl-N-(2-methoxyäthyl)-2-methylanilin
N-Chloracetyl-N-(2-methoxy-2-methyläthyl)-2,6-dimethylanilin
N-(2-Äthoxy-2-methyläthyl)-N-chloracetyl-2-äthyl-6-methylanilin
N-Chloracetyl-N-(1-äthyl-2-methoxyäthyl)-2,6-dimethylanilin
N-Chloracetyl-N-(2-methoxyäthyl)-2-methoxy-6-methylanilin
N-n-Butoxymethyl-N-chloracetyl-2-tert.-butylanilin
N-(2-Äthoxyäthyl-1-methyläthyl)-2,6-dimethylanilin
N-Chloracetyl-N-(2-methoxyäthyl)-2-chlor-6-methylanilin
N-(2-Äthoxyäthyl)-N-chloracetyl-2-chlor-6-methylanilin
N-(2-Äthoxyäthyl)-N-chloracetyl-2,3,6-trimethylanilin
N-Chloracetyl-1-(2-methoxyäthyl)-2,3,6-trimethylanilin
N-Chloracetyl-N-cyanomethyl-2,6-dimethylanilin
N-But-3-in-1-yl-N-chloracetylanilin
N-Chloracetyl-N-propargyl-2-äthyl-6-methylanilin
N-Chloracetyl-N-(1,3-dioxolan-2-ylmethyl)-2,6-dimethylanilin
N-Chloracetyl-N-(1,3-dioxolan-2-ylmethyl)-2-äthyl-6-methylanilin
N-Chloracetyl-N-(1,3-dioxan-2-ylmethyl)-2-äthyl-6-methylanilin
N-Chloracetyl-N-(2-furanylmethyl)-2,6-dimethylanilin
N-Chloracetyl-N-(2-furanylmethyl)-2-äthyl-6-methylanilin
N-Chloracetyl-N-(2-tetrahydrofuranylmethyl)-2,6-dimethylanilin
N-Chloracetyl-N-(N-propargylcarbamoylmethyl)-2,6-dimethylanilin
N-Chloracetyl-N-(N,N-dimethylcarbamoylmethyl)-2,6-dimethylanilin
N-(n-Butoxymethyl)-N-chloracetyl-2,6-diäthylanilin
N-(2-n-Butoxyäthyl)-N-chloracetyl-2,6-diäthylanilin
N-Chloracetyl-N-(2-methoxy-1,2-dimethyläthyl)-2,6-dimethylanilin
N-Chloracetyl-N-isopropyl-2,3-dimethylanilin
N-Chloracetyl-N-isopropyl-2-chloranilin
N-Chloracetyl-N-(1H-pyrazol-1-ylmethyl)-2,6-dimethylanilin
N-Chloracetyl-N-(1H-pyrazol-1-ylmethyl)-2-äthyl-6-methylanilin
N-Chloracetyl-N-(1H-1,2,4-triazol-1-ylmethyl)-2,6-dimethylanilin
N-Chloracetyl-N-(1H-1,2,4-triazol-1-ylmethyl)-2,6-diäthylanilin
N-Benzoylmethyl-N-chloracetyl-2,6-dimethylanilin
N-Benzoylmethyl-N-chloracetyl-2-äthyl-6-methylanilin
N-Chloracetyl-N-(5-methyl-1,3,4-oxadiazol-2-yl)-2,6-diäthylanilin
N-Chloracetyl-N-(5-methyl-1,3,4-oxadiazol-2-yl)-2-äthyl-6-methylanilin
N-Chloracetyl-N-(5-methyl-1,3,4-oxadiazol-2-yl)-2-tert.-butylanilin
N-Chloracetyl-N-(4-chlorbenzoylmethyl)-2,6-dimethylanilin
N-Chloracetyl-N-(1-methyl-5-methylthio-1,3,4-triazol-2-ylmethyl)-2,6-diäthylanilin

Weitere Halogenacetanilide, deren schädigende Wirkung auf Kulturpflanzen durch die neuen Oximäther der Formel I aufgehoben werden kann, sind in R. Wegler, „Chemie der Pflanzenschutz- und Schädlingsbekämpfungsmittel", Bd. 8, S. 90-93 und 322-327, aufgeführt.

Herbizid wirksame Thiolcarbamate, vor deren phytotoxischen Wirkung Kulturpflanzen durch die neuen Oximäther der Formel I geschützt werden können, sind ebenfalls bereits in grosser Zahl bekannt geworden (vgl. z.B. US-PS Nrn. 2913327, 3037853, 3175897, 3185720, 3198786, 3582314 und 3846115). Die Schutzwirkung der neuen Oximäther der Formel I lässt sich insbesondere beim Einsatz von Thiolcarbamaten in Getreide, Reis oder veredelter Sorghum-Hirse vorteilhaft ausnützen.

Die Thiolcarbamate, vor deren phytotoxischer Wirkung Kulturpflanzen, wie Getreide, Reis und veredelte Sorghum-Hirse bevorzugt geschützt werden können, entsprechen den allgemeinen Formeln XV und XVI

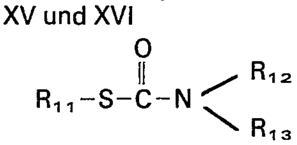
(XV)

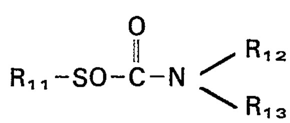
(XVI)

In diesen Formeln bedeutet $R_{11}$ niederes Alkyl, Alkenyl, Chlorallyl, Dichlorallyl, Trichlorallyl, Benzyl oder 4-Chlorbenzyl, $R_{12}$ $C_2$-$C_4$-Alkyl und $R_{13}$ $C_2$-$C_4$-Alkyl oder Cyclohexyl, wobei die Reste $R_{12}$ und $R_{13}$ zusammen mit dem Stickstoffatom, an das sie gebunden sind, einen Hexahydro-1H-azepin-, Dekahydrochinolin- oder 2-Methyldekahydrochinolinring bilden können.

Als einzelne Vertreter solcher Thiolcarbamate seien beispielsweise die folgenden genannt:

S-Äthyl-N,N-dipropylthiocarbamat
S-Äthyl-N,N-diisobutylthiocarbamat
S-2,3-Dichlorallyl-N,N-diisopropylthiolcarbamat
S-Propyl-N-butyl-N-äthylthiolcarbamat
S-2,3,3-Trichlorallyl-N,N-diisopropylthiocarbamat
S-Propyl-N,N-dipropylthiolcarbamat
S-Äthyl-X-äthyl-N-cyclohexylthiolcarbamat
S-Äthyl-N-hexahydro-1H-azepin-1-carbothioat
S-Isopropyl-N,N-hexamethylenthiolcarbamat
S-(p-Chlorbenzyl)-N,N-diäthylthiolcarbamat
N-Äthylthiocarbonyl-cis-decahydrochinolin
N-Propylthiocarbonyldecahydrochinaldin
S-Äthyl-N,N-bis-(n-butyl)thiolcarbamat
S-tert.-Butyl-N,N-bis-(n-propyl)thiolcarbamat

Neben den Chloracetanilid- und Thiolcarbamaten kommen auch Herbizide anderer Stoffklassen in Betracht, wie beispielsweise:

*Triazine und Triazinone:*

2,4-Bis(isopropylamino)-6-methylamino)-6-methylthio-1,3,5-triazin (Prometryn), 2,4-Bis-(äthylamino)-6-methylthio-1,3,5-triazin (Simetryn), 2-(1',2'-Dimethylpropylamino)-4-äthylamino-6-methylthio-1,3,5-triazin (Dimethametryn), 4-Amino-6-tert.-butyl-4,5-dihydro-3-methylthio-1,2,4-triazin-5-on (Metribuzin), 2-Chlor-4-äthylamino-6-isopropylamino-1,3,5-triazin (Atrazin), 2-Chlor-4,6-bis(äthylamino)-1,3,5-triazin (Simazin), 2-tert.-Butylamino-4-chlor-6-äthylamino-1,3,5-triazin (Terbuthylazin), 2-tert.-Butylamino-4-äthylamino-6-methoxy-1,3,5-triazin (Terbumeton), 2-tert.-Butylamino-4-äthylamino-6-methylthio-1,3,5-triazin (Terbutryn), 2-Äthylamino-4-isopropylamino-6-methylthio-1,3,5-triazin (Ametryn);

*Harnstoffe:*

1-(Benzothiazol-2-yl)-1,3-dimethylharnstoff; Phenylharnstoffe wie beispielsweise 3-(3-Chlor-p-tolyl)-1,1-dimethylharnstoff (Chlortoluron), 1,1-Dimethyl-3-(α,α,α-trifluor-m-tolyl)harnstoff (Fluometuron), 3-(4-Brom-3-chlorphenyl)-1-methoxy-1-methylharnstoff (Chlorbromuron), 3-(4-Bromphenyl)-1-methoxy-1-methylharnstoff (Metobromuron), 3-(3,4-Dichlorphenyl)-1-methoxy-1-methylharnstoff (Linuron), 3-(4-Chlorphenyl)-1-methoxy-1-methylharnstoff (Monolinuron), 3-(3,4-Dichlorphenyl)-1,1-dimethylharnstoff (Diuron), 3-(4-Chlorphenyl)-1,1-dimethylharnstoff (Monuron), 3-(3-Chlor-4-methoxyphenyl)-1,1-dimethylharnstoff (Metoxuron); Sulfonylharnstoffe wie beispielsweise N-(2-Chlorphenylsulfonyl)-N'-(4-methoxy-6-methyl-1,3,5-triazin-2-yl)harnstoff, N-(2-Methoxycarbonylphenylsulfonyl)-N'-(4,6-dimethylpyrimidin-2-yl)harnstoff, N-(2,5-Dichlorphenylsulfonyl)-N'-(4,6-dimethoxypyrimidin-2-yl)harnstoff, N-[2-(2-Butenyloxy)phenylsulfonyl]-N'-(4-methoxy-6-methyl-1,3,5-triazin-2-yl)harnstoff sowie die in den europäischen Patentpublikationen Nrn. 44808 und 44809 genannten Sulfonylharnstoffe;

*Chloracetamide:*

N-[1-Isopropyl-2-methylpropen-1-yl-(1)]-N-(2'-methoxyäthyl)chloracetamid;

*Diphenyläther und Nitrodiphenyläther:*

2,4-Dichlorphenyl-4'-nitrophenyläther (Nitrofen), 2-Chlor-1-(3'-äthoxy-4'-nitrophenoxy)-4-trifluormethylbenzol (Oxyfluorfen), 2',4'-Dichlorphenyl-3-methoxy-4-nitrophenyläther (Chlormethoxynil), 2-[4'-(2",4"-Dichlorphenoxy)phenoxy]propionsäuremethylester, N-(2'-Phenoxyäthyl)-2-[5'-(2"-chlor-4"-trifluormethylphenoxy)phenoxy]propionsäureamid, 2-[2-Nitro-5-(2-chlor-4-trifluormethylphenoxy)phenoxy]propionsäure-2-methoxyäthylester; 2-Chlor-4-trifluormethylphenyl-3'-oxazolin-2'-yl-4'-nitrophenyläther;

*Benzoesäurederivate:*

Methyl-5-(2',4'-dichlorphenoxy)-2-nitrobenzoat (Bifenox), 5-(2'-Chlor-4'-trifluormethylphenoxy)-2-nitrobenzoesäure (Acifluorfen), 2,6-Dichlorbenzonitril (Dichlobenil);

*Nitroaniline:*

2,6-Dinitro-N,N-dipropyl-4-trifluormethylanilin (Trifluralin), N-(1'-Äthylpropyl)-2,6-dinitro-3,4-xylidin (Pendimethalin);

*Oxadiazolone:*

5-tert.-Butyl-3-(2',4'-dichlor-5'-isopropoxyphenyl)-1,3,4-oxadiazol-2-on (Oxadiazon);

*Phosphate:*

S-2-Methylpiperidinocarbonylmethyl-O,O-dipropylphosphorodithioat (Piperophos);

*Pyrazole:*

1,3-Dimethyl-4-(2',4'-dichlorbenzoyl)-5-(4'-tolylsulfonyloxy)pyrazol.

Ferner *α-(Phenoxyphenoxy)propionsäurederivate* sowie *α-(Pyriyl-2-oxyphenoxy)propionsäurederivate.*

Die angewendete Menge der Gegenmittel schwankt zwischen etwa 0,01 und etwa 15 Gew.-Teilen pro Gewichtsteil Herbizid. Man ermittelt von Fall zu Fall, d.h. je nach verwendetem Herbizidtyp, welches Verhältnis in bezug auf optimale Wirkung bei der speziellen Kulturpflanze das geeignetste ist.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$-$C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Äther und Ester, wie Äthanol, Äthylenglykol, Äthylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonid oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen sind z.B. die Alkali-, Erdalkali- oder gegebenenfalls substituierten Ammoniumsalze von höheren Fettsäuren ($C_{10}$-$C_{22}$), wie z.B. die Na- oder K-Salze der Öl- oder Stearinsäure, oder von natürlichen Fettsäuregemischen, die z.B. aus Kokosnuss- oder Talgöl gewonnen werden können. Ferner sind auch die Fettsäuremethyltaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8-22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol/Äthylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure/Formaldehyd-Kondensationsproduktes.

Ferner kommen auch entsprechende Phosphate wie z.B. Salze des Phosphorsäureesters eines p-Nonylphenol-(4-14)/Äthylenoxyd-Adduktes in Frage.

Als nichtionisches Tensid kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6-18 Kohlenstoffatome im Alkylrest der Alkylphenole enthalten können.

Weitere geeignete nichtionische Tenside sind die wasserlöslichen, 20-250 Äthylenglykoläthergruppen und 10-1000 Propylenglykoläthergruppen enthaltenden Polyäthylenoxidaddukte an Polypropylenglykol, Äthylendiaminopolypropylenglykol und Alkylpolypropylenglykol mit 1-10 Kohlenstoffatomen in der Alkylkette. Die genannten Verbindungen enthalten üblicherweise pro Propylenglykoleinheit 1 bis 5 Äthylenglykoleinheiten.

Als Beispiele nichtionischer Tenside seien Nonylphenolpolyäthoxyäthanole, Ricinusölpolyglycoläther, Polypropylen/Polyäthylenoxy-Addukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8-22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Äthylsulfate vor, z.B. das Stearyltrimethylammoniumchlorid oder das Benzyldi-(2-chloräthyl)äthylammoniumbromid.

Die in der Formulierungstechnik gebräuchlichen Tenside sind u.a. in folgenden Publikationen beschrieben: „Mc Cutcheon's Detergents and Emulsifiers Annual", Mc Publishing Corp., Ringwood, New Jersey, 1979, Sisely and Wood, „Encyclopedia of Surface Active Agents", Chemical Publishing Co., Inc. New York, 1964.

Diese Zubereitungen enthalten in der Regel 0,1 bis 99%, insbesondere 0,1 bis 95%, Wirkstoff der Formel I, 1 bis 99% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

In den folgenden Beispielen sind die Temperaturen in Centigraden gegeben, die Druckangaben sind in Millibar und die Teile und Prozentangaben beziehen sich auf das Gewicht.

*Beispiel 1:*

*Herstellung von 1-(4-Chlorphenyl)-1-(1,3-dioxolan-2-ylmethoximino)-2,2,2-trifluoräthan (Verbindung Nr. 40)*

a) In einem 100-ml-Rundkolben werden 1,2 g (0,05 mol) metallisches Natrium in 50 ml absolutem Äthanol gelöst und 11,2 g (0,05 mol) 1-(4-Chlorphenyl)-1-hydroximino-2,2,2-trifluoräthan zugegeben. Nach beendigter Zugabe wird zunächst ½ h bei Raumtemperatur nachgerührt und dann das Lösungsmittel abgedampft. Zu der Lösung des Rückstandes in 50 ml Dimethylsulfoxid werden unter Rühren 8,4 g (0,05 mol) 2-Brommethyl-1,3-dioxolan zugetropft und 4 h bei 60-70° Innentemperatur nachgerührt. Danach wird die entstandene Suspension abgekühlt und auf eine Eis/Wasser-Mischung gegossen. Aus der erhaltenen Mischung wird das Reaktionsprodukt durch Extraktion mit Methylenchlorid gewonnen. Der Extrakt wird über Natriumsulfat getrocknet und das Lösungsmittel abgedampft. Als Rohprodukt wird ein orangefarbenes Öl erhalten, das durch Destillation im Hochvakuum gereinigt wird. Es werden so 12,5 g (80,6% der Theorie) 1-(4-Chlorphenyl)-1-(1,3-dioxolan-2-ylmethoximino)-2,2,2-trifluoräthan als farbloses Öl vom Siedepunkt 105-108°/0,1 mbar erhalten.

b) Das als Ausgangsmaterial benötigte 1-(4-Chlorphenyl)-1-hydroximino-2,2,2-trifluoräthan wird wie folgt hergestellt:

Eine Mischung von 417 g (2,0 mol) 4-Chlor-ω,ω,ω-trifluoracetophenon, 139 g (2,0 mol) Hydroxylaminhydrochlorid und 500 ml Pyridin wird 15 h bei Raumtemperatur gerührt. Danach wird das Pyridin abdestilliert und der ölige Rückstand in Eiswasser gerührt. Nach Extraktion mit Methylenchlorid, Trocknen des Extraktes über Natriumsulfat und Abdampfen des Lösungsmittels erhält man 438 g (98% der Theorie) 1-(4-Chlorphenyl)-1-hydroxyimino-2,2,2-trifluoräthan als Öl.

*Beispiel 2:*

*Herstellung von 1-Phenyl-1-(1,3-dioxolan-2-ylmethoximino)-2,2,2-trifluoräthan (Verbindung Nr. 38)*

In eine Lösung von 1,5 g (0,07 mol) metallischem Natrium in 50 ml absolutem Äthanol werden 13,8 g (0,07 mol) 1-(Hydroximino)-1-phenyl-2,2,2-trifluoräthan eingerührt und das Lösungsmittel abgedampft. In die Lösung des Rückstandes in 50 ml Dimethylsulfoxid werden unter Rühren 6,7 g (0,10 mol) 2-Brommethyl-1,3-dioxolan eingetropft. Nach beendigter Zugabe des 2-Brommethyl-1,3-dioxolans wird zunächst 4 h bei 60-70° Innentemperatur nachgerührt und dann die erhaltene Suspension auf ein Eis/Wasser-Gemisch gegossen. Nach Extraktion mit Methylenchlorid, Trocknen des Extraktes mit Natriumsulfat und Abdampfen des Lösungsmittels wird als Rohprodukt ein oranges Öl erhalten, das durch Destillation im Hochvakuum gereinigt wird. Es werden so 15,0 g (78% der Theorie) 1-Phenyl-1-(1,3-dioxolan-2-ylmethoximino)-2,2,2-trifluoräthan vom Siedepunkt 89-90°/0,1 mbar erhalten.

*Beispiel 3:*

*Herstellung von 1-(4-Chlorphenyl)-1-(2,2-diäthoxyäthoximino)-2,2,2-trifluoräthan (Verbindung Nr. 11).*

In eine Lösung von 2,3 g (0,1 mol) metallischem Natrium in 50 ml absolutem Äthanol werden 25,6 g (0,1 mol) 1-Hydroximino-1-(4-chlorphenyl)-2,2,2-trifluoräthan eingetropft. Nach beendigter Zugabe wird ½ h bei Raumtemperatur gerührt und dann das Lösungsmittel abgedampft. Der Lösung des Rückstandes in 50 ml Dimethylsulfoxid wird unter Rühren tropfenweise 29,7 g (0,15 mol) 2-Bromacetaldehyddiäthylacetal zugesetzt. Nach beendigter Zugabe wird 4 h bei 60-70° Innentemperatur gerührt und anschliessend das Reaktionsgemisch auf ein Eis/Wasser-Gemisch gegossen. Das durch Extraktion mit Methylenchlorid, Trocknen des Extrakts mit Natriumsulfat und Abdampfen des Lösungsmittels gewonnene ölige Rohprodukt wird durch Destillation im Hochvakuum gereinigt. Man erhält so 20,5 g (73,5% der Theorie) 1-(4-Chlorphenyl)-1-(2,2-diäthoxyäthoximino)-2,2,2-trifluoräthan vom Siedepunkt 95-97°/0,02 mbar als farbloses Öl.

*Beispiel 4:*

*Herstellung von 1-(4-Chlorphenyl)-1-(2,2-dimethoxyäthoximino)-2,2,2-trifluoräthan (Verbindung Nr. 2)*

In eine Lösung von 2,3 g (0,1 mol) metallischem Natrium in 50 ml absolutem Methanol werden 25,6 g (0,1 mol) 1-Hydroximino-1-(4-chlorphenyl)-2,2,2-trifluoräthan eingetropft. Nach beendigter Zugabe wird ½ h bei Raumtemperatur nachgerührt und dann das Lösungsmittel abgedampft. Zur Lösung des Rückstandes in 50 ml Dimethylsulfoxid wird unter Rühren eine Lösung von 33,8 g (0,2 mol) 2-Bromacetaldehyddimethylacetal in 60 ml Dimethylsulfoxid tropfenweise zugesetzt. Nach beendigter Zugabe wird 4 h bei 60-70° Innentemperatur nachgerührt. Dann wird das Reaktionsgemisch abgekühlt und auf ein Eis/Wasser-Gemisch gegossen. Das durch Extraktion mit Methylenchlorid, Trocknen des Extrakts mit Natriumsulfat und Abdampfen des Lösungsmittels gewonnene Rohprodukt wird durch Hochvakumdestillation gereinigt. Es werden so 22,0 g (70,5% der Theorie) 1-(4-Chlorphenyl)-1-(2,2-dimethoxy-äthoximino)-2,2,2-trifluoräthan vom Siedepunkt 96-98°/0,02 mbar als farbloses Öl erhalten.

Gemäss diesen Beispielen werden folgende Verbindungen hergestellt

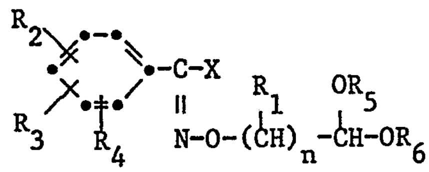

*Tabelle 1*

| Nr. | X | n | $R_1$ | $R_2$ | $R_3$ | $R_4$ | $R_5$ | $R_6$ | |
|---|---|---|---|---|---|---|---|---|---|
| 1 | $CF_3$ | 1 | H | H | H | H | $CH_3$ | | Sdp. 66-68°/ 0,03 mbar |
| 2 | $CF_3$ | 1 | H | H | 4-Cl | H | $CH_3$ | | Sdp. 96-98°/ 0,02 mbar |
| 3 | $CF_3$ | 1 | H | H | H | H | $C_2H_5$ | | Sdp. 77-78°/ 0,04 mbar |
| 4 | $CF_3$ | 1 | H | H | 4-F | H | $CH_3$ | | Sdp. 67-68°/ 0,01 mbar |
| 5 | $CF_3$ | 1 | H | H | 4-Cl | H | n-$C_3H_7$ | | |
| 6 | $CClF_2$ | 1 | H | H | 4-$OCH_3$ | H | n-$C_4H_9$ | | |
| 7 | $CClF_2$ | 2 | H | H | H | H | $C_2H_5$ | | |
| 8 | $CF_3$ | 1 | $CH_3$ | H | 3-$NO_2$ | H | $CH_3$ | | |
| 9 | $C_2F_5$ | 1 | $CH_3$ | 3-Cl | 4-Cl | H | $C_2H_5$ | | |
| 10 | $CClF_2$ | 1 | $CH_3$ | H | 2-Cl | H | $C_2H_5$ | | |
| 11 | $CF_3$ | 1 | H | H | 4-Cl | H | $C_2H_5$ | | Sdp. 95-97°/ 0,02 mbar |
| 12 | $CF_3$ | 1 | $CH_3$ | H | 3-$NO_2$ | H | $C_2H_5$ | | |
| 13 | $C_2F_5$ | 1 | $CH_3$ | H | 4-$OCH_3$ | H | $CH_3$ | | |
| 14 | $C_2F_5$ | 1 | $CH_3$ | H | 4-$OCF_3$ | H | i-$C_3H_7$ | | |
| 15 | $CF_3$ | 1 | H | H | 2-Cl | H | i-$C_3H_7$ | | |
| 16 | $CF_3$ | 1 | $CH_3$ | H | H | H | $CH_2-CH=CH_2$ | | |
| 17 | $C_2F_5$ | 1 | H | H | 4-Cl | H | $CH_3$ | | |
| 18 | $CF_3$ | 1 | H | H | 4-$OCH_3$ | H | $C_2H_5$ | | |
| 19 | $CF_3$ | 1 | H | H | 4-$OCF_3$ | H | n-$C_3H_7$ | | |
| 20 | $CF_3$ | 1 | $CH_3$ | H | 4-$SO_2$-$CF_3$ | H | $CH_3$ | | |
| 21 | $CClF_2$ | 1 | H | H | 4-Cl | H | $C_2H_5$ | | |
| 22 | $CF_3$ | 1 | $CH_3$ | H | 3-$CF_3$ | H | $CH_3$ | | |
| 23 | $C_2F_5$ | 1 | H | H | 4-$OCH_3$ | H | $CH_2-CH=CH_2$ | | |
| 24 | $C_3G_7$ | 1 | H | H | 4-Cl | H | $C_2H_5$ | | |
| 25 | $CF_3$ | 1 | $CH_3$ | H | 3-$CF_3$ | H | n-$C_3H_7$ | | |
| 26 | $CF_3$ | 1 | $C_2H_5$ | H | 3-$NO_2$ | H | $C_2H_5$ | | |
| 27 | $CF_3$ | 2 | H | H | 2-F | H | $CH_3$ | | |
| 28 | $CF_3$ | 1 | i-$C_3H_7$ | H | 2-Cl | H | $C_2H_5$ | | |
| 29 | $CF_3$ | 2 | $CH_3$ | H | 3-$CF_3$ | H | $C_2H_5$ | | |
| 30 | $CF_3$ | 1 | H | H | 3-$NO_2$ | H | $C_2H_5$ | | |
| 31 | $CF_3$ | 1 | H | H | 4-$OCH_3$ | H | $CH_2-CH=CH_2$ | | |
| 32 | $CF_3$ | 1 | H | H | 4-$SO_2CF_3$ | H | $CH_3$ | | |
| 33 | $C_2F_5$ | 1 | H | H | 3-$CF_3$ | H | $CH_3$<br>$CH_2-C=CH_2$ | | |
| 34 | $CF_3$ | 1 | H | | 4-Cl | H | $CH_2-CH=CH_2$ | | |
| 35 | $C_2F_5$ | 1 | H | H | 4-Cl | H | $CH_3$ | | |
| 36 | $C_2F_5$ | 1 | H | H | H | H | $CH_2-CH=CH_2$ | | |
| 37 | $CF_3$ | 1 | H | H | H | H | $CH_2-CH=CH_2$ | | |
| 38 | $CF_3$ | 1 | H | H | H | H | $-CH_2-CH_2-$ | | Sdp. 89-90°/ 0,1 mbar |
| 39 | $CF_3$ | 2 | H | H | H | H | $-CH_2-CH_2-$ | | Sdp. 92-93°/ 0,03 mbar |
| 40 | $CF_3$ | 1 | H | H | 4-Cl | H | $-CH_2-CH_2-$ | | Sdp.105-108°/ 0,1 mbar |
| 41 | $CF_3$ | 1 | H | H | 4-F | H | $-CH_2-CH_2-$ | | Sdp. 77-78°/ 0,02 mbar |

*Tabelle 1* (Fortsetzung)

| Nr. | X | n | $R_1$ | $R_2$ | $R_3$ | $R_4$ | $R_5$ | $R_6$ | |
|---|---|---|---|---|---|---|---|---|---|
| 42 | $CF_3$ | 2 | H | H | 4-F | H | $-CH_2-CH_2-$ | | Sdp. 78-79°/ 0,15 mbar |
| 43 | $CF_3$ | 1 | H | H | H | H | 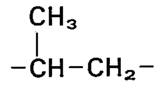 | | Sdp. 77-78°/ 0,05 mbar |
| 44 | $CF_3$ | 1 | H | H | H | H | 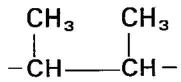 | | Sdp. 82-83°/ 0,07 mbar |
| 45 | $CF_3$ | 1 | H | H | 4-Cl | H | 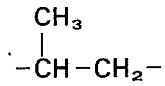 | | |
| 46 | $CClF_2$ | 1 | H | H | $4-OCH_3$ | H | 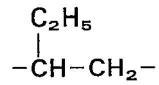 | | |
| 47 | $CF_3$ | 1 | H | 3-Cl | 4-Cl | H | $-CH_2-CH_2-$ | | |
| 48 | $CClF_2$ | 1 | H | H | H | H | $-CH_2-CH_2-$ | | |
| 49 | $C_2F_5$ | 1 | H | H | $4-OCF_3$ | H | $-CH_2-CH_2-$ | | |
| 50 | $CF_3$ | 1 | H | H | $4-OCF_3$ | H | $-CH_2-CH_2-$ | | |
| 51 | $CF_3$ | 1 | H | H | $3-CF_3$ | H | 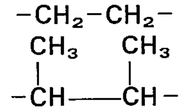 | | |
| 52 | $CF_3$ | 1 | H | H | $4-OCH_3$ | H | $-CH_2-CH_2-$ | | |
| 53 | $CF_3$ | 1 | H | H | $3-NO_2$ | H | 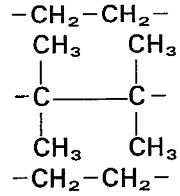 | | |
| 54 | $CClF_2$ | 1 | $CH_3$ | H | 2-F | H | $-CH_2-CH_2-$ | | |
| 55 | $C_2F_5$ | 1 | $CH_3$ | H | 2-F | H | $-CH_2-CH_2-$ | | |
| 56 | $CClF_2$ | 1 | $CH_3$ | H | 2-F | H | 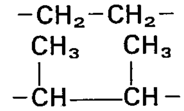 | | |
| 57 | $CF_3$ | 1 | H | 2-Cl | 4-Cl | H | $-CH_2-CH_2-$ | | |
| 58 | $CF_3$ | 1 | H | 2-Cl | 4-Cl | $5-NO_2$ | $-CH_2-CH_2-$ | | |
| 59 | $CF_3$ | 1 | H | $3-CH_3$ | $4-CH_3$ | H | $-CH_2-CH_2-$ | | |
| 60 | $CClF_2$ | 1 | $CH_3$ | $3-CH_3$ | 4-Cl | H | $-CH_2-CH_2-$ | | |
| 61 | $CF_3$ | 1 | $CH_3$ | 2-Cl | 4-Cl | $5-CF_3$ | $-CH_2-CH_2-$ | | |
| 62 | $CF_3$ | 1 | H | 2-F | 4-F | $5-NO_2$ | $-CH_2-CH_2-$ | | |
| 63 | $CF_3$ | 1 | H | H | H | H | $-CH_2-CH_2-CH_2-$ | | Sdp. 82-83°/ 0,04 mbar |
| 64 | $CF_3$ | 2 | H | H | H | H | $-CH_2-CH_2-CH_2-$ | | |
| 65 | $CF_3$ | 1 | H | H | 4-Cl | H | $-CH_2-CH_2-CH_2-$ | | Sdp. 96-98°/ 0,08 mbar |
| 66 | $CF_3$ | 1 | H | H | 4-F | H | $-CH_2-CH_2-CH_2-$ | | Sdp. 87-89°/ 0,04 mbar |
| 67 | $CF_3$ | 1 | H | H | H | H | 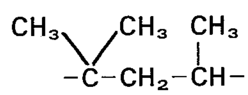 | | |
| 68 | $C_2F_5$ | 1 | H | H | $4-OCF_3$ | H | 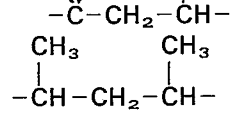 | | |
| 69 | $CClF_2$ | 1 | H | H | $4-OCH_3$ | H | 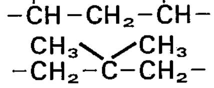 | | |
| 70 | $CClF_2$ | 1 | H | H | 4-Cl | H | 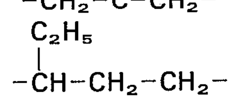 | | |
| 71 | $CF_3$ | 1 | $CH_3$ | H | $3-NO_2$ | H | $-CH_2-CH_2-CH_2-$ | | |
| 72 | $CF_3$ | 1 | $CH_3$ | H | $3-CF_3$ | H | $-CH_2-CH_2-CH_2-$ | | |
| 73 | $CF_3$ | 1 | $CH_3$ | 3-Cl | 4-Cl | H | $-CH_2-CH_2-CH_2-$ | | |

*Tabelle 1* (Fortsetzung)

| Nr. | X | n | $R_1$ | $R_2$ | $R_3$ | $R_4$ | $R_5$ $R_6$ | |
|---|---|---|---|---|---|---|---|---|
| 74 | $CF_3$ | 1 | H | 3-$CH_3$ | 4-$CH_3$ | H | $CH_3$ $CH_3$ $CH_3$ $CH_3$<br>$-C-CH_2-C-$ | |
| 75 | $C_2F_5$ | 1 | H | 3-Cl | 4-Cl | H | $-CH_2-CH_2-CH_2-$ | |
| 76 | $C_3F_7$ | 1 | H | H | H | H | $-CH_2-CH_2-CH_2-$ | |
| 77 | $CF_3$ | 1 | $CH_3$ | H | 4-$CH_3$ | H | $-CH_2-CH_2-CH_2-$ | |
| 78 | $CF_3$ | 1 | H | 2-Cl | 4-Cl | 5-$NO_2$ | $-CH_2-CH_2-CH_2-$ | |
| 79 | $CF_3$ | 1 | $CH_3$ | 2-Cl | 4-Cl | 5-$CF_3$ | $-CH_2-CH_2-CH_2-$ | |
| 80 | $CClF_2$ | 1 | $CH_3$ | H | H | H | $-CH_2-CH_2-CH_2-$ | |
| 81 | $CF_3$ | 2 | $CH_3$ | H | 4-Cl | H | $CH_3$ $CH_3$<br>$-CH_2-C-CH_2-$ | |
| 82 | $C_2F_5$ | 2 | H | H | 4-$CH_3$ | H | $-CH_2-CH_2-CH_2-$ | |
| 83 | $CF_3$ | 2 | H | 3-Cl | 4-Cl | H | $-CH_2-CH_2-CH_2-$ | |
| 84 | $CF_3$ | 1 | $CH_3$ | 3-$NO_2$ | 4-Cl | H | $-CH_2-CH_2-CH_2-$ | |
| 85 | $CF_3$ | 1 | $CH_3$ | H | 3-$NO_2$ | H | $C_2H_5$<br>$-CH-CH_2-CH_2-$ | |
| 86 | $CF_3$ | 1 | H | H | 2-Cl | H | $-CH_2-CH_2-CH_2-$ | |
| 87 | $CF_3$ | 1 | H | H | 2-F | H | $CH_3$ $CH_3$<br>$-CH_2-C-CH_2-$ | |
| 88 | $CClF_2$ | 1 | H | H | 4-$OCF_3$ | H | $-CH_2-CH_2-CH_2-$ | |
| 89 | $CF_3$ | 1 | H | | 4-$SO_2CF_3$ | H | $-CH_2-CH_2-CH_2-$ | |
| 90 | $CF_3$ | 1 | H | H | 4-Cl | H | $-CH(CH_3)CH_2-CH(CH_3)-$ | Sdp. 104-105°/ 0,04 mbar |
| 91 | $CF_3$ | 2 | H | H | 3-$CF_3$ | H | $-CH_2-CH_2-$ | |
| 92 | $CF_3$ | 1 | H | H | 3-$CF_3$ | H | $CH_3$ | |
| 93 | $CF_3$ | 1 | H | H | 3-$CF_3$ | H | $C_2H_5$ | |
| 94 | $C_2F_5$ | 1 | H | H | H | H | $CH_3$ | |
| 95 | $C_2F_5$ | 1 | H | H | H | H | $C_2H_5$ | |
| 96 | $C_2F_5$ | 1 | H | H | H | H | $-CH_2-CH_2$ | Sdp. 128-130°/ 0,1 mbar |
| 97 | $C_2F_5$ | 2 | H | H | H | H | $-CH_2-CH_2$ | |
| 98 | $C_2F_5$ | 1 | H | H | 4-Cl | H | $C_2H_5$ | |
| 99 | $C_2F_5$ | 1 | H | H | 4-Cl | H | $-CH_2-CH_2-$ | |
| 100 | $C_2F_5$ | 2 | H | H | 4-Cl | H | $-CH_2-CH_2-$ | |
| 101 | $C_2F_5$ | 1 | H | H | 4-F | H | $CH_3$ | |
| 102 | $C_2F_5$ | 1 | H | H | 4-F | H | $C_2H_5$ | |
| 103 | $C_2F_5$ | 1 | H | H | 4-F | H | $-CH_2-CH_2-$ | |
| 104 | $C_2F_5$ | 2 | H | H | 4-F | H | $-CH_2-CH_2-$ | |
| 105 | $C_2F_5$ | 1 | H | H | 2-F | H | $CH_3$ | |
| 106 | $C_2F_5$ | 1 | H | H | 2-F | H | $C_2H_5$ | |
| 107 | $C_2F_5$ | 1 | H | H | 2-F | H | $-CH_2-CH_2-$ | |
| 108 | $C_2F_5$ | 2 | H | H | 2-F | H | $-CH_2-CH_2-$ | |
| 109 | $CF_3$ | 1 | H | H | 2-F | H | $CH_3$ | |
| 110 | $CF_3$ | 1 | H | H | 2-F | H | $C_2H_5$ | |
| 111 | $CF_3$ | 2 | H | H | 2-F | H | $-CH_2-CH_2-$ | |
| 112 | $CF_3$ | 2 | H | H | 2-F | H | $-CH_2-CH_2-$ | |
| 113 | $CF_3$ | 1 | H | H | 2-F | H | $-CH_2-CH_2-CH_2-$ | |
| 114 | $CF_3$ | 1 | H | H | 3-$CF_3$ | H | $-CH(CH_3)-CH_2-$ | Sdp. 70-71°/ 0,08 mbar |
| 115 | $CF_3$ | 1 | H | H | 4-F | H | $-CH(CH_3)-CH_2-$ | Sdp. 80-81°/ 0,08 mbar |
| 116 | $CF_3$ | 1 | H | H | 3-$CF_3$ | H | $-CH_2-CH_2-$ | Sdp. 70-72°/ 0,04 mbar |
| 117 | $CF_3$ | 1 | H | 3-$CH_3$ | 4-$CH_3$ | H | $-CH_2-CH_2-$ | Sdp. 92-94°/ 0,02 mbar |
| 118 | $CF_3$ | 2 | H | H | 4-Cl | H | $-CH_2-CH_2-$ | Sdp. 110-114°/ 0,04 mbar |

*Tabelle 1* (Fortsetzung)

| Nr. | X | n | $R_1$ | $R_2$ | $R_3$ | $R_4$ | $R_5$ | $R_6$ | |
|---|---|---|---|---|---|---|---|---|---|
| 119 | $CF_3$ | 1 | H | H | 4-F | H | $-C_2H_5$ | | Sdp. 80-82°/ 0,4 mbar |
| 120 | $C_2H_5$ | 1 | H | H | H | H | $-CH(CH_3)-CH_2-$ | | Sdp. 87-90°/ 0,13 mbar |
| 121 | $C_2F_5$ | 1 | H | H | H | H | $-CH(CH_3)-CH_2-$ | | Sdp. 78-90°/ 0,15 mbar |
| 122 | $CF_3$ | 1 | H | H | H | H | $-CH(CH_3)-CH_2-CH_2-$ | | Sdp. 91-93°/ 0,08 mbar |

| Nr. | X | n | $R_1$ | $R_2$ | $R_3$ | $R_4$ | $R_5$ | $R_6$ | |
|---|---|---|---|---|---|---|---|---|---|
| 123 | $CF_3$ | 1 | H | H | H | H | $-CH(CH_3)-CH_2-$ | | Sdp. 85-86°/ 0,05 mbar |
| 124 | $CF_3$ | 1 | H | H | 4-Cl | H | $-CH(CH_3)-CH_2-CH_2-$ | | Sdp. 97-99°/ 0,08 mbar |
| 125 | $CF_3$ | 1 | H | H | 4-Cl | H | $-CH(CH_3)-CH_2-$ | | Sdp. 94-96°/ 0,05 mbar |
| 126 | $CF_3$ | 1 | H | H | 3-$CF_3$ | H | $-CH(CH_3)-CH_2$ | | Sdp. 80-81°/ 0,08 mbar |
| 127 | $CF_3$ | 1 | H | H | 4-F | H | $-CH(CH_3)-CH_2$ | | Sdp. 82-84°/ 0,12 mbar |
| 128 | $CF_3$ | 1 | H | H | H | H | $-CH(C_2H_5)-CH_2-$ | | Sdp. 80-82°/ 0,03 mbar |
| 129 | $CF_3$ | 1 | H | H | 4-Cl | H | $-CH(CH_3)-CH(CH_3)-$ | | Sdp. 97-99°/ 0,15 mbar |
| 130 | $CF_3$ | 1 | H | H | 3-$CF_3$ | H | $-CH_2-CH_2-CH_2$ | | Sdp. 88-90°/ 0,15 mbar |
| 131 | $CF_3$ | 1 | H | H | H | H | $-CH_2-CH_2-CH_2-CH_2-$ | | Sdp. 93-94°/ 0,09 mbar |
| 132 | $CF_3$ | 1 | H | H | H | H | $-CH_2CH=CH-CH_2$ | | Sdp. 92-93°/ 0,05 mbar |
| 133 | $CF_3$ | 1 | H | H | 2-Cl | H | $-CH_2-CH_2-$ | | Sdp. 91-92°/ 0,09 mbar |
| 134 | $CF_3$ | 1 | H | H | 4-$CH_3$ | H | $-CH_2-CH_2-$ | | Sdp. 82-83°/ 0,04 mbar |
| 135 | $CF_3$ | 1 | H | H | 4-Cl | H | $-CH_2CH=CH-CH_2-$ | | Sdp.107-109°/ 0,08 mbar |
| 136 | $CF_3$ | 1 | H | H | 4-Cl | H | $-CH_2-C(CH_3)_2-CH_2-$ | | Sdp.104-106°/ 0,09 mbar |
| 137 | $CF_3$ | 1 | H | H | 4-$CH_3$ | H | $-CH_2-C(CH_3)_2-CH_2-$ | | |
| 138 | $CF_3$ | 1 | H | H | 2-Cl | H | $-CH_2-C(CH_3)_2-CH_2-$ | | |
| 139 | $CF_3$ | 1 | H | H | 4-$CH_3$ | H | $-CH(CH_2)-CH_2-$ | | |
| 140 | $CF_3$ | 1 | H | H | 4-$CH_3$ | H | $-CH_2-CH_2-CH_2-$ | | |
| 141 | $CF_3$ | 1 | H | H | 4-$CH_3$ | H | $CH_3$ | | |
| 142 | $CF_3$ | 1 | H | H | 4-$CH_3$ | H | $C_2H_5$ | | |
| 143 | $CF_3$ | 2 | H | H | 4-$CH_3$ | H | $-CH_2-CH_2-$ | | |
| 144 | $CF_3$ | 2 | H | H | 2-Cl | H | $-CH_2-CH_2-$ | | |
| 145 | $CF_3$ | 2 | H | H | 4-$CH_3$ | H | $-CH_2-CH_2-CH_2-$ | | |
| 146 | $CF_3$ | 2 | H | H | 4-Cl | H | $-CH_2-CH_2-CH_2-$ | | |
| 147 | $CF_3$ | 2 | H | H | H | H | $-CH_2-CH_2-CH_2-$ | | |

| Nr. | X | n | $R_1$ | $R_2$ | $R_3$ | $R_4$ | $R_5$ | $R_6$ |
|---|---|---|---|---|---|---|---|---|
| 148 | $CF_3$ | 2 | H | H | 2-Cl | H | $-CH_2-CH_2-CH_2-$ | |
| 149 | $CF_3$ | 2 | H | H | 3-$CH_3$ | H | $-CH_2-CH_2-CH_2-$ | |
| 150 | $CF_3$ | 2 | H | H | 3-$CH_3$ | H | $-CH_2-CH_2-CH_2-$ | |
| 151 | $CF_3$ | 2 | H | H | H | H | $-CH_2-CH_2-$ | |

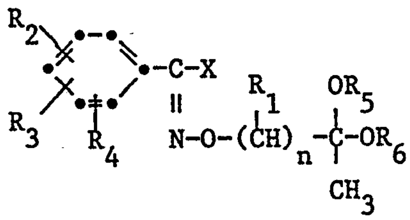

| Nr. | X | n | $R_1$ | $R_2$ | $R_3$ | $R_4$ | $R_5$ | $R_6$ |
|---|---|---|---|---|---|---|---|---|
| 161 | $CF_3$ | 1 | H | H | H | H | $-CH_2-CH_2$ | |
| 162 | $CF_3$ | 1 | H | H | 4-Cl | H | $-CH_2-CH_2-$ | |

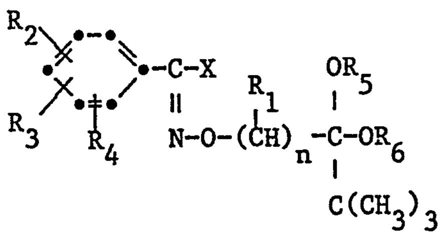

| Nr. | X | n | $R_1$ | $R_2$ | $R_3$ | $R_4$ | $R_5$ | $R_6$ |
|---|---|---|---|---|---|---|---|---|
| 171 | | 1 | H | H | H | H | $-CH_2-CH_2$ | |
| 172 | | 1 | H | H | 4-Cl | H | $-CH_2-CH_2$ | |

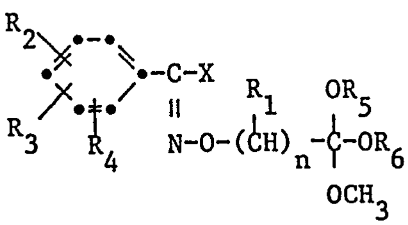

| Nr. | X | n | $R_1$ | $R_2$ | $R_3$ | $R_4$ | $R_5$ | $R_6$ |
|---|---|---|---|---|---|---|---|---|
| 181 | $CF_3$ | 1 | H | H | H | H | $CH_3$ | |
| 182 | $CF_3$ | 1 | H | H | H | H | $C_2H_5$ | |
| 183 | $CF_3$ | 1 | H | H | H | H | $-CH_2-CH_2-$ | |
| 184 | $CF_3$ | 1 | H | H | 4-Cl | H | $-CH_2-CH_2-$ | |
| 185 | $CF_3$ | 1 | H | H | 4-Cl | H | $C_2H_5$ | |

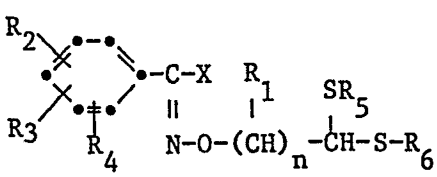

| Nr. | X | n | $R_1$ | $R_2$ | $R_3$ | $R_4$ | $R_5$ | $R_6$ |
|---|---|---|---|---|---|---|---|---|
| 186 | $CF_3$ | 1 | H | H | H | H | $-CH_2-CH_2$ | |
| 187 | $CF_3$ | 1 | H | H | 4-Cl | H | $-CH_2-CH_2$ | |
| 188 | $CF_3$ | 1 | H | H | $4-CH_3$ | H | $-CH_2-CH_2$ | |
| 189 | $CF_3$ | 1 | H | H | 4-F | H | $-CH_2-CH_2-$ | |
| 190 | $CF_3$ | 1 | H | H | H | H | $-CH_2-CH_2-CH_2-$ | |
| 191 | $CF_3$ | 1 | H | H | 4-Cl | H | $-CH_2-CH_2-CH_2-$ | |

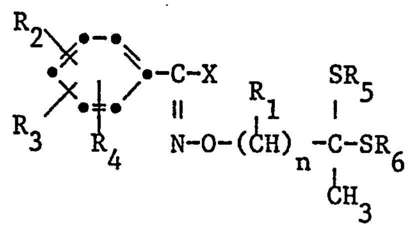

| Nr. | X | n | $R_1$ | $R_2$ | $R_3$ | $R_4$ | $R_5$ | $R_6$ |
|---|---|---|---|---|---|---|---|---|
| 192 | $CF_3$ | 1 | H | H | H | H | $-CH_2-CH_2-$ | |
| 193 | $CF_3$ | 1 | H | H | 4-Cl | H | $-CH_2-CH_2$ | |
| 194 | $CF_3$ | 1 | H | H | 4-$CH_3$ | H | $-CH_2-CH_2$ | |
| 195 | $CF_3$ | 1 | H | H | 4-F | H | $-CH_2-CH_2-$ | |
| 196 | $CF_3$ | 1 | H | H | H | H | $-CH_2-CH_2-CH_2-$ | |
| 197 | $CF_3$ | 1 | H | H | 4-Cl | H | $-CH_2-CH_2-CH_2-$ | |

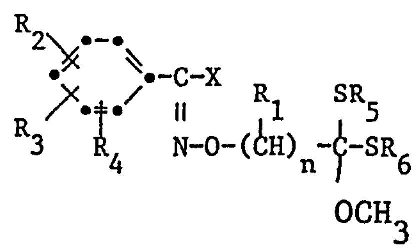

| Nr. | X | n | $R_1$ | $R_2$ | $R_3$ | $R_4$ | $R_5$ | $R_6$ |
|---|---|---|---|---|---|---|---|---|
| 201 | $CF_3$ | 1 | H | H | H | H | $-CH_2-CH_2-$ | |
| 202 | $CF_3$ | 1 | H | H | 4-Cl | H | $-CH_2-CH_2-$ | |
| 203 | $CF_3$ | 1 | H | H | 4-$CH_3$ | H | $-CH_2-CH_2-$ | |
| 204 | $CF_3$ | 1 | H | H | 4-F | H | $-CH_2-CH_2-$ | |

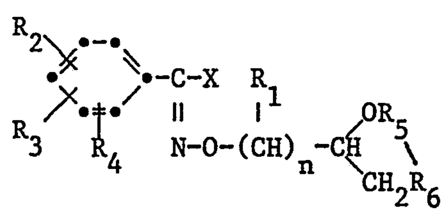

| Nr. | X | n | $R_1$ | $R_2$ | $R_3$ | $R_4$ | $R_5$ | $R_6$ | |
|---|---|---|---|---|---|---|---|---|---|
| 211 | $CF_3$ | 1 | H | H | H | H | 2-Furanyl | | |
| 212 | $CF_3$ | 1 | H | H | 4-Cl | H | 2-Furanyl | | Sdp.89-99°/ 0,07 mbar |
| 213 | $CF_3$ | 1 | H | H | 4-F | H | 2-Furanyl | | |
| 214 | $CF_3$ | 1 | H | H | 4-$CH_3$ | H | 2-Furanyl | | |
| 215 | $CF_3$ | 1 | H | H | 2-Cl | H | 2-Furanyl | | |
| 216 | $CF_3$ | 1 | H | 3-$CH_3$ | 4-$CH_3$ | H | 2-Furanyl | | |
| 217 | $CF_3$ | 1 | H | H | 3-$CF_3$ | H | 2-Furanyl | | |
| 218 | $CF_3$ | 1 | H | H | H | H | 2-Furanyl | | |
| 219 | $CF_3$ | 1 | H | H | H | H | 2-Pyranyl | | |
| 220 | $CF_3$ | 1 | H | H | 4-Cl | H | 2-Pyranyl | | |
| 221 | $CF_3$ | 1 | H | H | 4-$CH_3$ | H | 2-Pyranyl | | |

*Beispiel 5:*

*Formulierungsbeispiele für Wirkstoffe der Formel I oder Mischungen dieser Wirkstoffe mit Herbiziden*

| a) *Spritzpulver* (%) | a) | b) | c) |
|---|---|---|---|
| Wirkstoff der Formel I oder Mischung mit Herbizid | 20 | 60 | 0,5 |
| Na-ligninsulfonat | 5 | 5 | 5 |
| Na-laurylsulfat | 3 | — | — |
| Na-diisobutylnaphthalinsulfonat | — | 6 | 6 |
| Octylphenolpolyäthylenglykoläther (6-8 mol ÄO) | — | 2 | 2 |
| Hochdisperse Kieselsäure | 5 | 27 | 27 |
| Kaolin | 67 | — | — |
| Natriumchlorid | — | — | 59,5 |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) *Emulsionkonzentrat* (%) | a) | b) |
|---|---|---|
| Wirkstoff der Formel I oder Mischung mit Herbizid | 10 | 1 |
| Octylphenolpolyäthylenglykoläther (4-5 mol ÄO) | 3 | 3 |
| Ca-dodecylbenzolsulfonat | 3 | 3 |
| Ricinusölpolyglykoläther (36 mol ÄO) | 4 | 4 |
| Cyclohexanon | 30 | 10 |
| Xylolgemisch | 50 | 79 |

Aus diesem Konzentrat können durch Verdünnen mit Wasser Emulsionen jeder gewünschten Konzentration hergestellt werden.

| c) *Stäubemittel* (%) | a) | b) |
|---|---|---|
| Wirkstoff der Formel I oder Mischung mit Herbizid | 0,1 | 1 |
| Talkum | 99,9 | — |
| Kaolin | — | 99 |

Man erhält anwendungsfertige Stäubemittel, indem der Wirkstoff mit dem Träger vermischt und auf einer geeigneten Mühle vermahlen wird.

| d) *Extruder Granulat* (%) | a) | b) |
|---|---|---|
| Wirkstoff der Formel I oder Mischung mit Herbizid | 10 | 1 |
| Na-ligninsulfonat | 2 | 2 |
| Carboxymethylcellulose | 1 | 1 |
| Kaolin | 87 | 96 |

Der Wirkstoff wird mit den Zusatzstoffen vermischt, vermahlen und mit Wasser angefeuchtet. Dieses Gemisch wird extrudiert und anschliessend im Luftstrom getrocknet.

| e) *Umhüllungsgranulat* (%) | |
|---|---|
| Wirkstoff der Formel I oder Mischung mit Herbizid | 3 |
| Polyäthylenglykol (MG 200) | 3 |
| Kaolin | 94 |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungsgranulate.

| f) *Suspensionskonzentrat* (%) | a) | b) |
|---|---|---|
| Wirkstoff der Formel I oder Mischung mit Herbizid | 40 | 5 |
| Äthylenglykol | 10 | 10 |
| Nonylphenolpolyäthylenglykoläther (15 mol ÄO) | 6 | 1 |
| Na-ligninsulfonat | 10 | 5 |
| Carboxymethylcellulose | 1 | 1 |
| 37%ige wässerige Formaldehydlösung | 0,2 | 0,2 |
| Silikonöl in Form einer 75%igen wässerigen Emulsion | 0,8 | 0,8 |
| Wasser | 32 | 77 |

Der fein gemahlene Wirkstoff wird mit den Zusatzstoffen innig vermischt. Man erhält so ein Suspensionskonzentrat, aus welchem durch Verdünnen mit Wasser Suspensionen jeder gewünschten Konzentration hergestellt werden können.

| g) *Salzlösung* (%) | |
|---|---|
| Wirkstoff der Formel I oder Mischung mit Herbizid | 5 |
| Isopropylamin | 1 |
| Octylphenolpolyäthylenglykoläther (78 mol ÄO) | 3 |
| Wasser | 91 |

*Biologische Beispiele*

Die Fähigkeit der Verbindungen der Formel I, Kulturpflanzen vor der phytotoxischen Wirkung starker Herbizide zu schützen, kann aus den folgenden Beispielen ersehen werden. In den Versuchsbeschreibungen werden die Verbindungen der Formel I als Antidote (Gegenmittel) bezeichnet.

*Beispiel 6:*

*Versuch mit Antidote und Herbizid in Mais. Applikation von Antidote und Herbizid als Tankmischung-ppi im Vorauflauf.*

Die als Safener zu prüfende Substanz wird zusammen mit dem Herbizid auf die Erde gespritzt und dann eingemischt. Anschliessend werden Plastikcontainer (25 cm lang × 17 cm breit × 12 cm hoch) mit der behandelten Erde gefüllt. Maissamen der Sorte LG 5 werden eingesät. 21 d nach der Applikation wird die Schutzwirkung des Safeners in Prozent bonitiert. Als Referenz dienen dabei die mit Herbizid allein behandelten Pflanzen sowie die vollständig unbehandelte Kontrolle. Eine gute (signifikante) Safenerwirkung ist erreicht, wenn die Phytotoxizität von schwerer bis mittlerer Pflanzenschädigung zu leichten, reversiblen Schäden oder zu gänzlicher Verträglichkeit verringert werden kann. Dazu genügt im allgemeinen eine Schutzwirkung von mindestens 25%. Die Resultate sind untenstehend zusammengefasst.

*Herbizid:*

S-Äthyldipropylthiocarbamat, EPTC

*Antidote:*

1-(4-Chlorphenyl)-1-(3,5-dioxolan-2-yl-methoximino)-2,2,2-trifluoräthan, Verbindung Nr. 40

| Aufwandmenge (kg/ha) | | Schutzwirkung |
|---|---|---|
| Herbizid | Antidote | (%) |
| 8 | – | 0 |
| 8 | 8 | 38 |
| 8 | 4 | 50 |
| 8 | 2 | 75 |
| 4 | – | 0 |
| 4 | 4 | 50 |
| 4 | 2 | 75 |
| 4 | 1 | 75 |

*Beispiel 7:*

*Vesuch mit Antidote und Herbizid in Sorghum im Vorauflaufverfahren. Applikation der Antidote durch Samenbeizung*

Sorghum-Samen der Sorte Funk G 522 werden mit der als Safener zu prüfenden Substanz in einem Glasbehälter gemischt. Samen und Produkt werden durch Schütteln und Rotation des Behälters gut zusammengemischt. Anschliessend werden Plastikcontainer (25 cm lang × 17 cm breit × 12 cm hoch) mit sandiger Lehmerde gefüllt und die gebeizten Samen eingesät. Nach dem Bedecken des Samens wird das Herbizid auf die Bodenoberfläche gesprüht. 19 d nach der Applikation wird die Schutzwirkung des Safeners in Prozent bonitiert. Als Referenz dienen dabei die mit Herbizid allein behandelten Pflanzen sowie die vollständig unbehandelte Kontrolle. Die Resultate sind untenstehend zusammengefasst.

*Herbizid:*

N-Chloracetyl-N-(2-methoxy-1-methyl-äthyl)-2-äthyl-6-methylanilin (Metolachlor)

*Antidote:*

1-(4-Chlorphenyl)-1-(3,5-dioxolan-2-yl-methoxymino)-2,2,2-trifluoräthan, Verbindung Nr. 40

| Aufwandmenge (kg/ha) | | Schutzwirkung |
|---|---|---|
| Herbizid | Antidote | (%) |
| 4 | 0 | 0 |
| 4 | 1 | 38 |
| 4 | 0,5 | 63 |
| 4 | 0,25 | 63 |
| 2 | 0 | 2 |
| 2 | 1 | 38 |
| 2 | 0,5 | 63 |
| 2 | 0,25 | 63 |
| 2 | 0,125 | 63 |
| 1 | 0 | 0 |
| 1 | 1 | 50 |
| 1 | 0,5 | 63 |
| 1 | 0,25 | 75 |
| 1 | 0,125 | 88 |

Andere Versuche ergeben folgende Werte:

| Aufwandmenge (kg/ha) | | Schutzwirkung |
|---|---|---|
| Herbizid | Antidote | (%) |
| 4 | 0 | 0 |
| 4 | 2 | 50 |
| 4 | 1 | 63 |
| 4 | 0,5 | 63 |

| Aufwandmenge (kg/ha) | | Schutzwirkung |
|---|---|---|
| Herbizid | Antidote | (%) |
| 2 | 0 | 0 |
| 2 | 2 | 50 |
| 2 | 1 | 63 |
| 2 | 0,5 | 63 |
| 1 | 0 | 0 |
| 1 | 2 | 50 |
| 1 | 1 | 63 |
| 1 | 0,5 | 63 |

| Aufwandmenge (kg/ha) | | Schutzwirkung |
|---|---|---|
| Herbizid | Antidote | (%) |
| 2 | 0 | 0 |
| 4 | 4 | 50 |
| 4 | 2 | 63 |
| 4 | 1 | 63 |
| 2 | 0 | 0 |
| 2 | 4 | 50 |
| 2 | 2 | 50 |
| 2 | 1 | 50 |
| 2 | 0,5 | 50 |
| 1 | 0 | 0 |
| 1 | 4 | 25 |
| 1 | 2 | 25 |
| 1 | 1 | 38 |
| 1 | 0,5 | 50 |

In einem weiteren Versuch wurde das Herbizid N-Chloracetyl-N-methoxymethyl-2,6-diäthylanilin, Alachlor und als Antidote der 1-(4-Chlorphenyl)-1-(1,3-dioxolan-2-ylmethoximino)-2,2,2-trifluoräthan (Verbindung Nr. 40) eingesetzt.

| Aufwandmenge (kg/ha) | | Schutzwirkung |
|---|---|---|
| Herbizid | Antidote | (%) |
| 4 | 0 | 0 |
| 4 | 2 | 63 |
| 4 | 1 | 75 |
| 2 | 0 | 0 |
| 2 | 4 | 50 |
| 2 | 2 | 63 |
| 2 | 1 | 75 |
| 2 | 0,5 | 75 |
| 1 | 0 | 0 |
| 1 | 4 | 38 |
| 1 | 2 | 50 |
| 1 | 1 | 63 |
| 1 | 0,5 | 63 |

*Beispiel 8:*

*Versuch mit Antidote und Herbizid in Sorghum. Applikation von Herbizid und Antidote als Tankmischung im Vorauflauf*

Plastikcontainer (25 cm lang×17 cm breit× 12 cm hoch) werden mit sandiger Lehmerde gefüllt und Sorghum-Samen der Sorte Funk G 522 eingesät. Nach dem Bedecken der Samen wird die als Safener zu prüfende Substanz zusammen mit dem Herbizid in verdünnte Lösung als Tankmischung auf die Bodenoberfläche gesprüht. 30 d nach der Applikation wird die Schutzwirkung des Safeners in Prozent bonitiert. Als Referenz dienen dabei die mit dem Herbizid allein behandelten Pflanzen sowie die vollständig unbehandelte Kontrolle.

Die Ergebnisse sind nachstehend zusammengefasst.

*Herbizid:*

N-Chloracetyl-N-(2-methoxy-1-methyl)-2-äthyl-6-methylanilin (Metolachlor)

*Antidote:*

1-(4-Chlorphenyl)-1-(1,3-dioxolan-2-yl-methoximino)-2,2,2-trifluoräthan, Verbindung Nr. 40

| Aufwandmenge (kg/ha) | | Schutzwirkung (%) |
|---|---|---|
| Herbizid | Antidote | |
| 4 | 0 | 0 |
| 4 | 4 | 63 |
| 4 | 2 | 63 |
| 2 | 0 | 0 |
| 2 | 2 | 63 |
| 2 | 1 | 63 |
| 1 | 0 | 0 |
| 1 | 1 | 50 |
| 1 | 0,5 | 50 |

In einem weiteren Versuch wurde als Herbizid N-Chloracetyl-N-methoxymethyl-2,6-diäthylanilin und als Antidote das 1-(4-Chlorphenyl)-1-(1,3-dioxolan-2-ylmethoximino)-2,2,2-trifluoräthan eingesetzt. Der Versuch wurde mit Hirsesamen Funk G 522 ohne Zusatz von Unkräutern durchgeführt.

| Aufwandmenge (kg/ha) | | Schutzwirkung (%) |
|---|---|---|
| Herbizid | Antidote | |
| 2 | 0 | 0 |
| 2 | 2 | 75 |
| 2 | 1 | 88 |
| 1 | 0 | 0 |
| 1 | 1 | 75 |
| 1 | 0,5 | 75 |

*Beispiel 9:*

*Versuch mit Antidote und Herbizid in Soja. Applikation von Herbizid und Antidote als Tankmischung im Vorauflauf*

Töpfe (oberer ⌀6 cm) werden mit sandiger Lehmerde gefüllt und Sojasamen der Sorte Hark werden eingesät. Nach dem Bedecken der Samen wird die als Safener zu prüfende Substanz zusammen mit dem Herbizid in verdünnte Lösung als Tankmischung auf die Bodenoberfläche versprüht. 21 d nach der Herbizidapplikation wird die Schutzwirkung des Safeners in Prozent bonitiert. Als Referenzen dienen dabei die mit Herbizid allein behandelten Pflanzen sowie die vollständig unbehandelte Kontrolle.

Die Ergebnisse sind wie folgt

*Herbizid:*

4-Amino-6-tert.-butyl-4,5-dihydro-3-methylthio-1,2,4-triazin-5-on Metribuzin

| Aufwandmenge (kg/ha) | | relative Schutzwirkung (%) |
|---|---|---|
| Antidote | Herbizid | |
| Nr. 1 1,5 | 0,75 | 25 |
| Nr. 2 1,5 | 0,75 | 25 |
| Nr. 11 1,5 | 0,75 | 25 |

*Beispiel 10:*

*Versuch mit Antidote und Herbizid in Weizen. Applikation von Herbizid und Antidote als Tankmischung im Nachauflauf*

Weizensamen der Sorte Farnese werden in Plastiktöpfe (oberer ⌀ 11 cm), die 0,5 l Erde enthalten, im Gewächshaus ausgesät. Nach dem Bedekken der Samen wird die als Safener zu prüfende Substanz zusammen mit dem Herbizid als Tankmischung im Nachauflauf appliziert. 20 d nach der Applikation wird die Schutzwirkung des Safeners in Prozent bonitiert. Als Referenzen dienen dabei die mit Herbizid allein behandelten Pflanzen sowie die vollständig unbehandelte Kontrolle.

Die Ergebnisse sind wie folgt

*Herbizid:*

α-[4-(2,4-Dichlorpyridyl-2-oxy)phenoxy]propionsäurepropargylester

| Aufwandmenge (kg/ha) | | relative Schutzwirkung (%) |
|---|---|---|
| Herbizid | Antidote | |
| Nr. 2 1,5 | 0,75 | 25 |
| Nr. 40 1,5 | 0,75 | 25 |
| Nr. 41 1,5 | 0,75 | 25 |
| Nr. 65 1,5 | 0,75 | 50 |

*Beispiel 11:*

*Versuch mit Antidote und Herbizid in Reis. Applikation der Antidote während der Samenquellung des Reises, Applikation des Herbizides auf den feuchten Boden im Vorauflauf*

Reissamen werden während 48 h mit Lösungen der als Safener zu prüfenden Substanz von 100 ppm getränkt. Anschliessend werden die Samen etwa 2 h trocknen gelassen, bis sie nicht mehr

kleben. Plastikcontainer (25 cm lang×17 cm breit×12 cm hoch) werden bis 2 cm unter dem Rand mit sandigem Lehm gefüllt. Die vorgequollenen Samen werden auf der Bodenoberfläche des Containers gesät und nur ganz schwach gedeckt. Die Erde wird in einem feuchten (nicht sumpfigen) Zustand gehalten. Dann wird das Herbizid in verdünnter Lösung auf die Bodenoberfläche versprüht. Der Wasserstand wird entsprechend dem Wachstum sukzessiv erhöht. 18 d nach der Herbizidapplikation wird die Schutzwirkung des Safeners in Prozent bonitiert. Als Referenz dienen dabei die mit dem Herbizid allein behandelten Pflanzen sowie die vollständig unbehandelte Kontrolle.

Das Ergebnis ist wie folgt

*Herbizid:*

2-Chlor-2',6'-diäthyl-N-(2"-propyloxyäthyl)-acetamid, Pretilachlor

| Aufwandmenge | | relative Schutzwirkung (%) |
|---|---|---|
| Antidote | Herbizid (kg/ha) | |
| Nr. 63 100 ppm | 0,25 | 25 |

*Beispiel 12:*

*Versuch mit Antidote und Herbizid in Reis. Applikation der Antidote als Saatbeize, Applikation des Herbizides auf den feuchten Boden im Vorauflauf*

Reissamen werden mit der als Safener zu prüfenden Substanz in einem Glasbehälter gemischt. Samen und Produkt werden durch Schütteln und Rotation gut zusammengemischt. Dann werden Container (47 cm lang×29 cm breit×24 cm hoch) mit sandiger Lehmerde gefüllt und die gebeizten Samen werden eingesät. Nach dem Bedecken des Samens wird das Herbizid in einer verdünnten Lösung auf die Bodenoberfläche versprüht. Etwa 20 d nach der Saat (3-Blattstadium der Reispflanzen) wird die Bodenoberfläche mit 4 cm Wasser Höhe beschichtet. 30 d nach der Herbizidapplikation wird die Schutzwirkung des Safeners in Prozent bonitiert. Als Referenzen dienen dabei die mit Herbizid allein behandelten Pflanzen sowie die vollständig unbehandelte Kontrolle.

Die Ergebnisse sind wie folgt

*Herbizid:*

2,6-Dinitro-N,N-dipropyl-4-trifluormethyl-anilin Trifluralin

| Aufwandmenge | | relative Schutzwirkung (%) |
|---|---|---|
| Antidote | Herbizid (kg/ha) | |
| Nr. 40 2 g/kg Samen | 2 | 25 |
| Nr. 40 2 g/kg Samen | 1 | 38 |

*Herbizid:*

α-[4-(2,4-Dichlorphenoxy)phenoxy]propionsäuremethylester Hoelon

| Aufwandmenge | | relative Schutzwirkung (%) |
|---|---|---|
| Antidote | Herbizid (kg/ha) | |
| Nr. 40 1 g/kg Samen | 1 | 50 |
| Nr. 40 2 g/kg Samen | 0,5 | 63 |
| Nr. 40 1 g/kg Samen | 1 | 25 |
| Nr. 40 1 g/kg Samen | 0,5 | 38 |

*Herbizid:*

α-[4-(2',4'-Dichlorpyridyl-2'-oxy)phenoxy] propionsäurepropargylester

| Aufwandmenge | | relative Schutzwirkung (%) |
|---|---|---|
| Antidote | Herbizid (kg/ha) | |
| Nr. 40 2 g/kg Samen | 0,5 | 50 |
| Nr. 40 2 g/kg Samen | 0,25 | |

**Patentansprüche**

1. Oximäther der Formel (I)

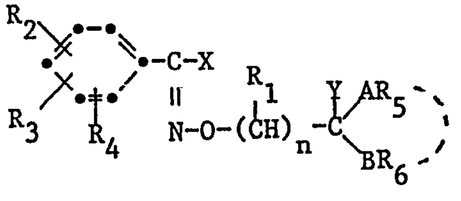

(I)

in welcher n 1 oder 2, $R_1$ und $R_2$ je Wasserstoff oder $C_1$-$C_4$-Alkyl, $R_3$ und $R_4$ je Wasserstoff, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_1$-$C_4$-Halogenalkylthio, $C_1$-$C_4$-Alkylsulfinyl, $C_1$-$C_4$-Alkylsulfonyl, $C_1$-$C_4$-Halogenalkylsulfinyl, $C_1$-$C_4$-Halogenalkylsulfonyl oder Nitro, $R_5$ und $R_6$ unabhängig voneinander je $C_1$-$C_4$-Alkyl, $C_1$-$C_4$-Halogenalkyl, $C_2$-$C_8$-Alkoxyalkyl, $C_3$-$C_4$-

Alkinyl oder zusammen eine 2- bis 6gliedrige Alkylen oder Alkenylenbrücke, die 1- bis 4mal durch $C_1$-$C_4$-Alkyl, $C_1$-$C_4$-Halogenalkyl, $C_1$-$C_4$-Alkoxy, $C_2$-$C_4$-Alkenyloxy, $C_2$-$C_8$-Alkoxyalkyl oder $C_3$-$C_8$-Alkenyloxyalkyl substituiert sein kann, A und B unabhängig voneinander je Sauerstoff, Schwefel oder eines davon die Methingruppe, X einen fluorierten $C_1$-$C_3$-Alkylrest, der zusätzlich auch Chlor enthalten kann, und Y Wasserstoff, $C_1$-$C_4$-Alkyl, $C_1$-$C_4$-Halogenalkyl, $C_1$-$C_4$-Alkoxy, $C_1$-$C_4$-Halogenalkoxy oder Phenyl bedeuten.

2. Oximäther der Formel (I) gemäss Anspruch 1, in denen A und B je Sauerstoff und Y Wasserstoff bedeuten, während n, $R_1$ bis $R_6$ und X die im Anspruch 1 gegebene Bedeutung haben.

3. Oximäther der Formel (I) gemäss Anspruch 1, in denen A und B je Schwefel und Y Wasserstoff bedeuten, während n, $R_1$ bis $R_6$ und X die im Anspruch 1 gegebene Bedeutung haben.

4. Oximäther der Formel (I) gemäss Anspruch 1, in denen A und B je Sauerstoff und Y $C_1$-$C_4$-Alkyl oder Phenyl bedeuten, während n, $R_1$ bis $R_6$ und X die im Anspruch 1 gegebene Bedeutung haben.

5. Oximäther der Formel (I) gemäss Anspruch 1, worin A und B je Schwefel, Y $C_1$-$C_4$-Alkyl oder Phenyl bedeuten, während n, $R_1$ bis $R_6$ und X die im Anspruch 1 gegebene Bedeutung haben.

6. Oximäther der Formel (I) gemäss Anspruch 1, worin A und B je Sauerstoff, Y $C_1$-$C_4$-Alkoxy bedeuten während n, $R_1$ bis $R_6$ und X die im Anspruch 1 gegebene Bedeutung haben.

7. Oximäther der Formel (I) gemäss Anspruch 1, worin A und B je Schwefel, Y $C_1$-$C_4$-Alkoxy bedeuten, während n, $R_1$ bis $R_6$ und X die im Anspruch 1 gegebene Bedeutung haben.

8. Oximäther der Formel (I) gemäss Anspruch 1, worin A Sauerstoff, B die Methingruppe und Y Wasserstoff bedeuten, während n, $R_1$ bis $R_6$ und X die im Anspruch 1 gegebene Bedeutung haben.

9. Oximäther der Formel (I) gemäss Anspruch 1, in welchen n für 1 steht, $R_1$, $R_2$ und $R_4$ Wasserstoff darstellen, $R_3$ in 4-Stellung steht und Wasserstoff, Halogen, Methyl, Difluormethyl, Trifluormethyl, Chlordifluormethyl, Methoxy, Difluormethoxy, Trifluormethoxy, Chlordifluormethoxy, Tetrafluoräthyl, Tetrafluoräthoxy oder Trifluormethylsulfonyl bedeutet, X perfluoriertes $C_1$-$C_3$-Alkyl oder Chlordifluormethyl ist, während A, B, $R_5$, $R_6$ und Y die im Anspruch 1 gegebene Bedeutung haben.

10. Oximäther der Formel (I) nach Anspruch 1, in welchen n für 1 steht, $R_1$, $R_2$ und $R_4$ Wasserstoff darstellen, $R_3$ in 4-Stellung steht und Wasserstoff, Fluor, Chlor, Methyl, Difluormethyl, Trifluormethyl, Chlordifluormethyl, Methoxy, Difluormethoxy, Trifluormethoxy, Chlordifluormethoxy, Tetrafluoräthyl, Tetrafluoräthoxy oder Trifluormethylsulfonyl bedeutet, X Trifluormethyl, Chlordifluormethyl oder Pentafluoräthyl ist und $R_5$ und $R_6$ zusammen eine gegebenenfalls durch 1-4-Methylgruppen substituierte 1,2-Äthylengruppe bedeuten, während A, B und Y die im Anspruch 1 gegebene Bedeutung haben.

11. Oximäther der Formel (I) gemäss Anspruch 1, in welchen n für 1 steht, $R_1$, $R_2$ und $R_4$ Wasserstoff darstellen, $R_3$ in 4-Stellung steht und Wasserstoff, Fluor, Chlor, Trifluormethyl oder Trifluormethoxy bedeutet, X Trifluormethyl, Chlordifluormethyl oder Pentafluoräthyl ist und $R_5$ und $R_6$ zusammen eine gegebenenfalls durch 1-4-Methylgruppen substituierte 1,2-Äthylengruppe darstellen.

12. Oximäther der Formel (I) gemäss Anspruch 1, in welchen n für 1 steht, $R_1$, $R_2$ und $R_4$ Wasserstoff darstellen, $R_3$ in 2-Stellung steht und Fluor oder Chlor bedeutet, X Trifluormethyl, Chlordifluormethyl oder Pentafluoräthyl ist und $R_5$ und $R_6$ zusammen eine gegebenenfalls durch 1-4-Methylgruppen substituierte 1,2-Äthylengruppe bedeuten.

13. Oximäther der Formel (I) gemäss Anspruch 1, in welchen n für 1 steht, $R_1$, $R_2$ und $R_4$ Wasserstoff darstellen, $R_3$ in 3-Stellung steht und Trifluormethyl oder Nitro bedeutet, X Trifluormethyl, Chlordifluormethyl oder Pentafluoräthyl ist und $R_5$ und $R_6$ zusammen eine gegebenenfalls durch 1-4-Methylgruppen substituierte 1,2-Äthylengruppe bedeuten.

14. 1-Phenyl-1-(1,3-dioxolan-2-ylmethoximino)-2,2,2-trifluoräthan gemäss Anspruch 1.

15. 1-(4-Chlorphenyl)-1-(1,3-dioxolan-2-ylmethoximino)-2,2,2-trifluoräthan gemäss Anspruch 1.

16. 1-(4-Chlorphenyl)-1-(2,2-diäthoxyäthoximino)-2,2,2-trifluoräthan gemäss Anspruch 1.

17. 1-(4-Chlorphenyl)-1-(2,2-dimethoxyäthoximino)-2,2,2-trifluoräthan gemäss Anspruch 1.

18. 1-(4-Fluorphenyl)-1-(1,3-dioxolan-2-ylmethoximino)-2,2,2-trifluoräthan gemäss Anspruch 1.

19. 1-(3,4-Dimethylphenyl)-1-(1,3-dioxolan-2-ylmethoximino)-2,2,2-trifluoräthan gemäss Anspruch 1.

20. 1-(Fluorphenyl)-1-(1,3-dioxolan-4-methyl-2-ylmethoximino)-2,2,2-trifluoräthan gemäss Anspruch 1.

21. 1-Phenyl-1-(1,3-dioxan-2-ylmethoximino)-2,2,2-trifluoräthan gemäss Anspruch 1.

22. 1-(3-Trifluormethylnaphthyl)-1-(1,3-dioxan-2-ylmethoximino)-2,2,2-trifluoräthan gemäss Anspruch 1.

23. 1-Phenyl-(1,3-dioxolan-2-ylmethoximino)-2,2,3,3,3-pentafluoräthan gemäss Anspruch 1.

24. 1-(4-Chlorphenyl)-1-(1,3-dioxolan-2-yläthyloximino)-2,2,2-trifluoräthan gemäss Anspruch 1.

25. 1-(4-Fluorphenyl)-1-(1,3-dioxolan-2-ylmethoximino)-2,2,2-trifluoräthan gemäss Anspruch 1.

26. 1-(4-Fluorphenyl)-1-(1,3-dioxolan-2-yläthylimino)-2,2,2-trifluoräthan gemäss Anspruch 1.

27. 1-(4-Chlorphenyl)-1-(1,3-dioxolan-4,5-dimethyl-2-ylmethoximino)-2,2,2-trifluoräthan gemäss Anspruch 1.

28. 1-(3-Trifluormethylphenyl)-1-(1,3-dioxolan-4-äthyl-2-ylmethoximino)-2,2,2-trifluoräthan gemäss Anspruch 1.

29. Verfahren zur Herstellung der Oximäther der Formel (I) gemäss Anspruch 1, dadurch gekennzeichnet, dass man ein Salz eines Oxims der Formel (VI)

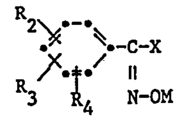

(VI)

in welcher M ein Alkalimetall- oder Erdalkalimetallkation darstellt und $R_2$, $R_3$, $R_4$ und X die im Anspruch 1 angegebene Bedeutung haben, mit einem Halogenacetal der Formel (VII)

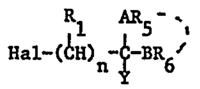

(VII)

in welcher Hal ein Halogenatom, insbesondere ein Chloratom oder ein Bromatom, bedeutet und A, B, n, $R_1$, $R_5$, $R_6$ und Y die im Anspruch 1 angegebene Bedeutung haben, umsetzt.

30. Verfahren nach Anspruch 29, dadurch gekennzeichnet, dass man als Salz eines Oxims der Formel (II) das Natrium- oder Kaliumsalz verwendet.

31. Verfahren zur Herstellung von Oximen der Formel I gemäss, Anspruch 1, in denen A und B je Sauerstoff oder Schwefel und Y $C_1$-$C_4$-Alkyl oder Phenyl bedeuten, während n, $R_1$ bis $R_6$ und X die im Anspruch 1 gegebene Bedeutung haben, dadurch gekennzeichnet, dass man in einem inerten Lösungsmittel oder Verdünnungsmittel einen Oximäther der Formel X

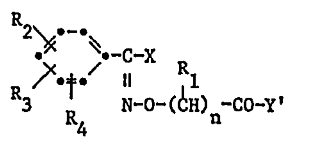

(X)

worin Y' $C_1$-$C_4$-Alkyl oder Phenyl bedeutet und n, $R_1$ bis $R_4$ und X die im Anspruch 1 gegebene Bedeutung haben, mit einem Ameisensäureorthoester der Formel (XI) umsetzt.

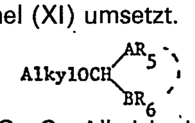

(XI)

worin Alkyl $C_1$-$C_6$-Alkyl bedeutet und A, B, $R_5$ und $R_6$ die im Anspruch 1 gegebene Bedeutung haben.

32. Verfahren zur Herstellung von Verbindungen der Formel (I) gemäss Anspruch 1, in denen A und B je Sauerstoff oder Schwefel, $R_5$ und $R_6$ zusammen eine 2- bis 6gliedrige Alkylen- oder Alkenylenbrücke, die durch $C_1$-$C_4$-Alkyl, $C_1$-$C_4$-Halogenalkyl, $C_1$-$C_4$-Alkoxy, $C_2$-$C_4$-Alkenyloxy, $C_2$-$C_8$-Alkoxyalkyl oder $C_3$-$C_8$-Alkenyloxyalkyl substituiert sein kann und Y $C_1$-$C_4$-Alkoxy bedeuten, während n, $R_1$ bis $R_4$ und X die im Anspruch 1 gegebene Bedeutung haben, dadurch gekennzeichnet, dass man einen Oximäther der Formel (XII)

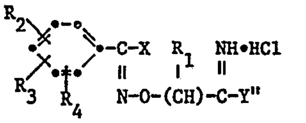

(XII)

worin Y" $C_1$-$C_4$-Alkoxy bedeutet, wobei n, R bis $R_4$ und X die im Anspruch 1 gegebene Bedeutung haben, in einem inerten Lösungs- oder Verdünnungsmittel mit einem Diol oder Dithiol der Formel (XIII) umsetzt.

$$HA-R_5-R_6-BH \quad (XIII)$$

worin A und B je Sauerstoff oder Schwefel und $R_5$ und $R_6$ zusammen eine 2- bis 6gliedrige Alkylen- oder Alkenylenbrücke bilden, die 1- bis 4mal durch $C_1$-$C_4$-Alkyl, $C_1$-$C_4$-Halogenalkyl, $C_1$-$C_4$-Alkoxy, $C_2$-$C_4$-Alkenyloxy, $C_2$-$C_8$-Alkoxyalkoxy oder $C_3$-$C_8$-Alkenyloxyalkyl substituiert sein kann.

33. Verfahren nach den Ansprüchen 29 bis 32, dadurch gekennzeichnet, dass man die Umsetzungen in Acetonitril, Dimethylformamid oder Dimethylsulfoxid als Lösungs- oder Verdünnungsmittel durchführt.

34. Mittel zum Schützen von Kulturpflanzen vor Schädigung durch Herbizide, dadurch gekennzeichnet, dass es neben inerten Träger- und Zusatzstoffen als wirksame Komponente einen Oximäther der Formel (I) gemäss Anspruch 1 enthält.

35. Mittel nach Anspruch 34, gekennzeichnet durch einen Gehalt an

a) einem herbizid wirksamen Halogenacetanilid oder einem herbizid wirksamen Thiolcarbamat, und

b) einem Oximäther der Formel (I) gemäss Anspruch 1 als Gegenmittel.

36. Mittel nach Anspruch 35, gekennzeichnet durch einen Gehalt an

a) einem Halogenacetanilid der Formel XIV

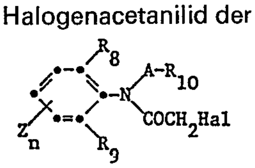

(XIV)

in welcher Hal Halogen, insbesondere Chlor oder Brom, $R_8$ und $R_9$ unabhängig voneinander je Wasserstoff, Halogen, niederes Alkyl, Alkoxy, Alkylthio, Halogenalkyl, Alkoxyalkyl oder Alkylthioalkyl, Z Wasserstoff, Halogen, niederes Alkyl, Alkoxy, Alkylthio, Halogenalkyl, Alkoxyalkyl oder Alkylthioalkyl, wobei die Reste Z vorzugsweise in 3-Stellung in bezug auf das Stickstoffatom stehen, n 0 bis 3, A Alkylen, insbesondere Methylen, 1,1- und 1,2-Äthylen, wobei 1,2-Äthylen durch 1 bis 2 niedere Alkylgruppen substituiert sein kann, und $R_{10}$ niederes Alkoxy, Hydroxycarbonyl, Alkoxycarbonyl, Carbamoyl, N-Alkylcarbamoyl, N,N-Dialkylcarbamoyl, Cyano, einen gegebenenfalls substituierten stickstoffhaltigen heterocyclischen Rest, Alkanoyl, gegebenenfalls substituiertes Benzyl, gegebenenfalls substituiertes 1,3,4-Oxadiazol-2-yl, 1,3,4-Thiadiazol-2-yl, 1,3,4-Triazol-3-yl oder 1,3,4-Triazol-1-yl bedeutet, und

b) einem Oximäther der Formel (I) gemäss Anspruch 1 als Gegenmittel.

37. Mittel nach Anspruch 35, gekennzeichnet durch einen Gehalt an

a) einem Thiolcarbamat der Formeln (XV) und (XVI)

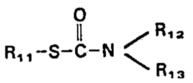 (XV) 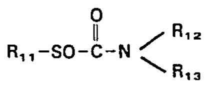 (XVI)

in welchen $R_{11}$ niederes Alkyl, Alkenyl, Chlorallyl, Dichlorallyl, Trichlorallyl, Benzyl, oder 4-Chlorbenzyl, $R_{12}$ $C_2$-$C_4$-Alkyl und $R_{13}$ $C_2$-$C_4$-Alkyl oder Cyclohexyl bedeutet, wobei die Reste $R_{12}$ und $R_{13}$ zusammen mit dem Stickstoffatom, an das sie gebunden sind, einen Hexahydro-1H-azepin, Dekahydrochinolin- oder 2-Methyldekahydrochinolinring bilden können, und

b) einem Oximäther der Formel I gemäss Anspruch 1 als Gegenmittel.

38. Verwendung der Oximäther der Formel (I) gemäss Anspruch 1 zum Schützen von Kulturpflanzen gegen die schädigende Wirkung von Herbiziden.

39. Verwendung der Oximäther der Formel (I) gemäss Anspruch 1 nach Anspruch 36 zum Schützen von Getreide-, Mais-, und Sorghum-Kulturen vor der Schädigung durch Halogenacetanilide der Formel (XIV) und Thiolcarbamaten der Formeln (XV) und (XVI) wie vorstehend definiert.

40. Verfahren zum Schützen von Kulturpflanzen vor Schäden, die bei der Applikation von Herbiziden auftreten, dadurch gekennzeichnet, dass man

a) die Anbaufläche für die Pflanze vor oder während der Applikation des Herbizids, oder

b) den Samen oder die Stecklinge der Pflanzen oder die Pflanze selbst mit einer wirksamen Menge eines Oximäthers der Formel I gemäss Anspruch 1 behandelt.

## Claims

1. An oxime ether of the Formula (I)

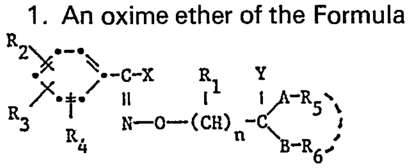 (I)

wherein n is 1 or 2, each of $R_1$ and $R_2$ is hydrogen or $C_1$-$C_4$ alkyl, each of $R_3$ and $R_4$ is hydrogen, halogen, $C_1$-$C_4$ alkyl, $C_1$-$C_4$ haloalkyl, $C_1$-$C_4$ alkoxy, $C_1$-$C_4$ haloalkoxy, $C_1$-$C_4$ alkylthio, $C_1$-$C_4$ haloalkylthio, $C_1$-$C_4$ alkylsulfinyl, $C_1$-$C_4$ alkylsulfonyl, $C_1$-$C_4$ haloalkylsulfinyl, $C_1$-$C_4$ haloalkylsulfonyl or nitro, each of $R_5$ and $R_6$ independently of the other is $C_1$-$C_4$ alkyl, $C_1$-$C_4$ haloalkyl, $C_2$-$C_8$ alkoxyalkyl, $C_3$-$C_4$ alkynyl, or together form a 2- to 6-membered alkylene or alkenylene bridge which may be substituted by 1 to 4 $C_1$-$C_4$ alkyl, $C_1$-$C_4$ haloalkyl, $C_1$-$C_4$ alkoxy, $C_2$-$C_4$ alkenyloxy, $C_2$-$C_8$ alkoxyalkyl or $C_3$-$C_8$ alkenyloxyalkyl groups, each of A and B independently of the other is oxygen or sulfur, or one of A and B is the methine group, X is a fluorinated $C_1$-$C_3$ alkyl radical which may also additionally contain chlorine, and Y is hydrogen, $C_1$-$C_4$ alkyl, $C_1$-$C_4$ haloalkyl, $C_1$-$C_4$ alkoxy, $C_1$-$C_4$ haloalkoxy or phenyl.

2. An oxime ether of the Formula (I) according to Claim 1, wherein each of A and B is oxygen, Y is hydrogen, and n, $R_1$ to $R_6$ and X are as defined in Claim 1.

3. An oxime ether of the Formula (I) according to Claim 1, wherein each of A and B is sulfur, Y is hydrogen, and n, $R_1$ to $R_6$ and X are as defined in Claim 1.

4. An oxime ether of the Formula (I) according to Claim 1, wherein each of A and B is oxygen, Y is $C_1$-$C_4$ alkyl or phenyl, and n, $R_1$ to $R_6$ and X are as defined in Claim 1.

5. An oxime ether of the Formula (I) according to Claim 1, wherein each of A and B is sulfur, Y is $C_1$-$C_4$ alkyl or phenyl, and n, $R_1$ to $R_6$ and X are as defined in Claim 1.

6. An oxime ether of the Formula (I) according to Claim 1, wherein each of A and B is oxygen, is $C_1$-$C_4$ alkoxy, and n, $R_1$ to $R_6$ and X are as defined in Claim 1.

7. An oxime ether of the Formula (I) according to Claim 1, wherein each of A and B is sulfur, Y is $C_1$-$C_4$ alkoxy, and n, $R_1$ to $R_6$ and X are as defined in Claim 1.

8. An oxime ether of the Formula (I) according to Claim 1, wherein A is oxygen, B is the methine group and Y is hydrogen, and n, $R_1$ to $R_6$ and X are as defined in Claim 1.

9. An oxime ether of the Formula (I) according to Claim 1, wherein n is 1, $R_1$, $R_2$ and $R_4$ are hydrogen, $R_3$ is in the 4-position and is hydrogen, halogen, methyl, difluoromethyl, trifluoromethyl, chlorodifluoromethyl, methoxy, difluoromethoxy, trifluoromethoxy, chlorodifluoromethoxy, tetrafluoroethyl, tetrafluoroethoxy or trifluoromethylsulfonyl, X is perfluorinated $C_1$-$C_3$ alkyl or chlorodifluoromethyl, and A, B, $R_5$, $R_6$ and Y are as defined in Claim 1.

10. An oxime ether of the Formula (I) according to Claim 1, wherein n is 1, $R_1$, $R_2$ and $R_4$ are hydrogen, $R_3$ is in the 4-position and is hydrogen, fluorine, chlorine, methyl, difluoromethyl, trifluoromethyl, chlorodifluoromethyl, methoxy, difluoromethoxy, trifluoromethoxy, chlorodifluoromethoxy, tetrafluoroethyl, tetrafluoroethoxy or trifluoromethylsulfonyl, X is trifluoromethyl, chlorodifluoromethyl or pentafluoroethyl, and $R_5$ und $R_6$ together are a 1,2-ethylene group which is unsubstituted or substituted by 1 to 4 methyl groups, and A, B and Y are as defined in Claim 1.

11. An oxime ether of the Formula (I) according to Claim 1, wherein n is 1, $R_1$, $R_2$ and $R_4$ are hydrogen, $R_3$ is in the 4-position and is hydrogen, fluorine, chlorine, trifluoromethyl or trifluoromethoxy, X is trifluoromethyl, chlorodifluoromethyl or pentafluoroethyl, and $R_5$ and $R_6$ together are a 1,2-ethylene group which is unsubstituted or substituted by 1 to 4 methyl groups.

12. An oxime ether of the Formula (I) according to Claim 1, wherein n is 1, $R_1$, $R_2$ and $R_4$ are hydrogen, $R_3$ is in the 2-position and is fluorine or chlorine, X is trifluoromethyl, chlorodifluoromethyl or pentafluoroethyl, and $R_5$ and $R_6$ together are a 1,2-ethylene group which is unsubstituted or substituted by 1 to 4 methyl groups.

13. An oxime ether of the Formule (I) according to Claim 1, wherein n is 1, $R_1$, $R_2$ and $R_4$ are

hydrogen, $R_3$ is in the 3-position and is trifluoromethyl or nitro, X is trifluoromethyl, chlorodifluoromethyl or pentafluoroethyl, and $R_5$ and $R_6$ together are a 1,2-ethylene group which is unsubstituted or substituted by 1 to 4 methyl groups.

14. 1-Phenyl-1-(1,3-dioxolan-2-ylmethoximino)-2,2,2-trifluoroethane according to Claim 1.

15. 1-(4-Chlorophenyl)-1-(1,3-dioxolan-2-ylmethoximino)-2,2,2-trifluoroethane according to Claim 1.

16. 1-(4-Chlorophenyl)-1-(2,2-diethoxyethoximino)-2,2,2-trifluoroethane according to Claim 1.

17. 1-(4-Chlorophenyl)-1-(2,2-dimethoxyethoximino)-2,2,2-trifluoroethane according to Claim 1.

18. 1-(4-Fluorophenyl)-1-(1,3-dioxolan-2-ylmethoximino)-2,2,2-trifluoroethane according to Claim 1.

19. 1-(3,4-Dimethylphenyl)-1-(1,3-dioxolan-2-ylmethoximino)-2,2,2-trifluoroethane according to Claim 1.

20. 1-(Fluorophenyl)-1-(1,3-dioxolan-4-methyl-2-ylmethoximino)-2,2,2-trifluoroethane according to Claim 1.

21. 1-Phenyl-1-(1,3-dioxan-2-ylmethoximino)-2,2,2-trifluoroethane according to Claim 1.

22. 1-(3-Trifluoromethylnaphthyl)-1-(1,3-dioxan-2-ylmethoximino)-2,2,2-trifluoroethane according to Claim 1.

23. 1-Phenyl-(1,3-dioxolan-2-ylmethoximino)-2,2,3,3,3-pentafluoroethane according to Claim 1.

24. 1-(4-Chlorophenyl)-1-(1,3-dioxolan-2-ylethyloximino)-2,2,2-trifluoroethane according to Claim 1.

25. 1-(4-Fluorophenyl)-1-(1,3-dioxolan-2-ylmethoxyimino)-2,2,2-trifluoroethane according to Claim 1.

26. 1-(4-Fluorophenyl)-1-(1,3-dioxolan-2-ylethylimino)-2,2,2-trifluoroethane according to Claim 1.

27. 1-(4-Chlorophenyl)-1-(1,3-dioxolan-4,5-dimethyl-2-ylmethoximino)-2,2,2-trifluoroethane according to Claim 1.

28. 1-(3-Trifluoromethylphenyl)-1-(1,3-dioxolan-4-ethyl-2-ylmethoximino)-2,2,2-trifluoroethane according to Claim 1.

29. A process for the preparation of an oxime ether of the Formula (I) according to Claim 1, which process comprises reacting a salt of an oxime of the Formula VI

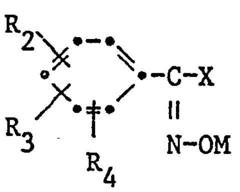

(VI)

wherein M is an alkali metal cation or an alkaline earth metal cation and $R_2$, $R_3$, $R_4$ and X are as defined in Claim 1, with a haloacetal of the Formula (VII)

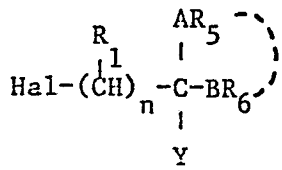

(VII)

wherein Hal is a halogen atom, preferably a chlorine atom or a bromine atom, and A, B, n, $R_1$, $R_5$, $R_6$ and Y are as defined in Claim 1.

30. A process according to Claim 29, wherein the sodium or potassium salt is used as salt of an oxime of the Formula II.

31. A process for the preparation of an oxime of the Formula (I) according to Claim 1, wherein each of A and B is oxygen or sulfur and Y is $C_1$-$C_4$ alkyl or phenyl, and n, $R_1$ to $R_6$ and X are as defined in Claim 1, which process comprises reacting an oxime ether of the formula (X)

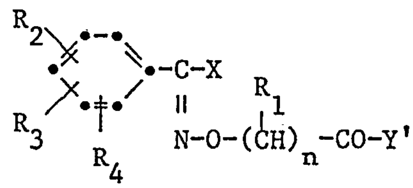

(X)

wherein Y' is $C_1$-$C_4$ alkyl or phenyl and n, $R_1$ to $R_4$ and X are as defined in Claim 1, with an orthoformate of the Formula (XI)

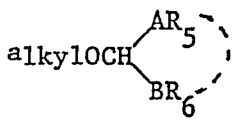

(XI)

wherein alkyl is $C_1$-$C_6$ alkyl and A, B, $R_5$ and $R_6$ are as defined in Claim 1, in an inert solvent or diluent.

32. A process for the preparation of a compound of the Formula I according to Claim 1, wherein each of A and B is oxygen or sulfur, $R_5$ and $R_6$ together form a 2- to 6-membered alkylene or alkenylene bridge which may be substituted by $C_1$-$C_4$ alkyl, $C_1$-$C_4$ haloalkyl, $C_1$-$C_4$ alkoxy, $C_2$-$C_4$ alkenyloxy, $C_2$-$C_8$ alkoxyalkyl or $C_3$-$C_8$ alkenyloxyalkyl, Y is $C_1$-$C_4$ alkoxy, and n, $R_1$ to $R_4$ and X are as defined in Claim 1, which process comprises reacting an oxime ether of the formula XII

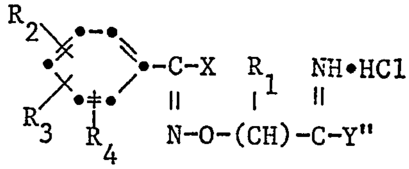

(XII)

wherein Y'' is $C_1$-$C_4$ alkoxy and n, $R_1$ to $R_4$ and X are as defined in Claim 1, in an inert solvent or diluent, with a diol or dithiol of the Formula (XIII)

$$HA-R_5-R_6-BH \quad (XIII)$$

wherein each of A and B is oxygen or sulfur and $R_5$ and $R_6$ together form a 2- to 6-membered alkylene or alkenylene bridge which may be substituted by

1 to 4 $C_1$-$C_4$ alkyl, $C_1$-$C_4$ haloalkyl, $C_1$-$C_4$ alkoxy, $C_2$-$C_4$ alkenyloxy, $C_2$-$C_8$ alkoxyalkoxy or $C_3$-$C_8$ alkenyloxyalkyl groups.

33. A process according to Claims 29 to 32, wherein the reaction is carried out in acetonitrile, dimethylformamide or dimethylsulfoxide as solvent or diluent.

34. A composition for protecting cultivated plants from damage caused by herbicides, which contains an oxime ether of the Formula (I) as active component, together with inert carriers and adjuvants.

35. A composition according to Claim 34, which contains

(*a*) a herbicidal haloacetanilide or a herbicidal thiocarbamate, and

(*b*) an oxime ether of the Formula (I) according to Claim 1 as antidote.

36. A composition according to Claim 35, which contains

(*a*) a haloacetanilide of the Formula (XIV)

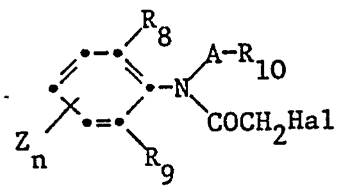

(XIV)

wherein Hal is halogen, preferably chlorine or bromine, each of $R_8$ and $R_9$ independently of the other is hydrogen, halogen, lower alkyl, alkoxy, alkylthio, haloalkyl, alkoxyalkyl or alkylthioalkyl, Z is hydrogen, halogen, lower alkyl, alkoxy, alkylthio, haloalkyl, alkoxyalkyl or alkylthioalkyl, which radicals Z are preferably in the 3-position with respect to the nitrogen atom, n is 0 to 3, A is alkylene, preferably methylene, 1,1-ethylene, and 1,2-ethylene which may be substituted by 1 or 2 lower alkyl groups, and $R_{10}$ is lower alkoxy, hydroxycarbonyl, alkoxycarbonyl, carbamoyl, N-alkylcarbamoyl, N,N-dialkylcarbamoyl, cyano, an unsubstituted or substituted nitrogen-containing heterocyclic radical, alkanoyl, unsubstituted or substituted benzyl, unsubstituted or substituted 1,3,4-oxadiazol-2-yl, 1,3,4-thiadiazol-2-yl, 1,3,4-triazol-3-yl or 1,3,4-triazol-1-yl, and

(*b*) an oxime ether of the Formula (I) according to Claim 1 as antidote.

37. A composition according to Claim 35, which contains

(*a*) a thiocarbamate of the Formulae (XV) and (XVI)

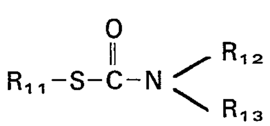

(XV)

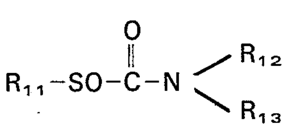

(XVI)

wherein $R_{11}$ is lower alkyl, alkenyl, chloroallyl, dichloroallyl, trichloroallyl, benzyl or 4-chlorobenzyl, $R_{12}$ is $C_2$-$C_4$ alkyl and $R_{13}$ is $C_2$-$C_4$ alkyl or cyclohexyl, and $R_{12}$ and $R_{13}$ together with the nitrogen atom to which they are attached can form a hexahydro-1H-azepine, decahydroquinoline or 2-methyldecahydroquinoline ring, and

(*b*) an oxime ether of the Formula I according to Claim 1 as antidote.

38. Use of an oxime ether of the Formula (I) according to Claim 1 for protecting cultivated plants from the harmful action of herbicides.

39. Use of an oxime ether of Formula (I) according to Claim 1 for protecting cereals, maize and sorghum crops from the damage caused by haloacetanilides of the Formula (XIV) and by thiocarbamates of the Formula XV or XVI as defined above.

40. A method of protecting cultivated plants from damage caused by the application of herbicides, which method comprises

(*a*) treating the locus of the plant before or during application of the herbicide, or

(*b*) treating the seeds or cuttings of the plants or the plant itself with an effective amount of an oxime ether of the Formula (I) according to Claim 1.

## Revendications

1. Ethers d'oximes de formule (I)

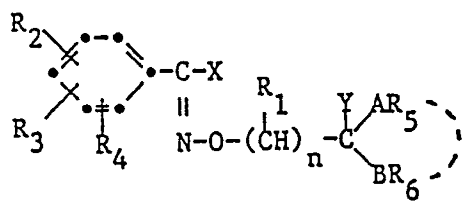

(I)

dans laquelle n est égal à 1 ou 2, $R_1$ et $R_2$ représentent chacun un atome d'hydrogène ou un groupe alkyle en $C_1$-$C_4$, $R_3$ et $R_4$ représentent chacun un atome d'hydrogène, d'halogène, un groupe alkyle en $C_1$-$C_4$, halogénoalkyle en $C_1$-$C_4$, alcoxy en $C_1$-$C_4$, halogénoalcoxy en $C_1$-$C_4$, alkylthio en $C_1$-$C_4$, halogénoalkylthio en $C_1$-$C_4$, alkylsulfinyle en $C_1$-$C_4$, alkylsulfonyle en $C_1$-$C_4$, halogénoalkylsulfinyle en $C_1$-$C_4$, halogénoalkylsulfonyle en $C_1$-$C_4$ ou nitro, $R_5$ et $R_6$ représentent chacun, indépendamment l'un de l'autre, un groupe alkyle en $C_1$-$C_4$, halogénoalkyle en $C_1$-$C_4$, alcoxyalkyle en $C_2$-$C_8$, alcynyle en $C_3$-$C_4$ ou forment ensemble un groupe alkylène ou un pont alcénylène contenant 2 à 6 chaînons et qui peut porter 1 à 4 substituants alkyle en $C_1$-$C_4$, halogénoalkyle en $C_1$-$C_4$, alcoxy en $C_1$-$C_4$, alcényloxy en $C_2$-$C_4$, alcoxyalkyle en $C_2$-$C_8$ ou alcénoyloxyalkyle en $C_3$-$C_8$, A et B représentent chacun, indépendamment l'un de l'autre, l'oxygène, le soufre, ou bien l'un de ces symboles représente le groupe méthine, X représente un groupe alkyle en $C_1$-$C_3$ fluoré et qui peut en outre contenir également du chlore, et Y représente l'hydrogène, un groupe alkyle en $C_1$-$C_4$, hagénoalkyle en $C_1$-$C_4$, alcoxy en $C_1$-$C_4$, halogénoalcoxy en $C_1$-$C_4$ ou phényle.

2. Ethers d'oximes de formule (I) selon la revendication 1, dans lesquels A et B représentent chacun l'oxygène et Y représente l'hydrogène, n, $R_1$ à $R_6$ et X ayant les significations indiquées dans la revendication 1.

3. Ethers d'oximes de formule (I) selon la revendication 1, dans lesquels A et B représentent chacun le soufre et Y l'hydrogène, n, $R_1$ à $R_6$ et X ayant les significations indiquées dans la revendication 1.

4. Ethers d'oximes de formule (I) selon la revendication 1, dans lesquels A et B représentent chacun l'oxygène et Y un groupe alkyle en $C_1$-$C_4$ ou phényle, n, $R_1$ à $R_6$ et X ayant les significations indiquées dans la revendication 1.

5. Ethers d'oximes de formule (I) selon la revendication 1, dans lesquels A et B représentent chacun le soufre, Y un groupe alkyle en $C_1$-$C_4$ ou phényle, n, $R_1$ à $R_6$ et X ayant les significations indiquées dans la revendication 1.

6. Ethers d'oximes de formule (I) selon la revendication 1, dans lesquels A et B représentent chacun l'oxygène, Y un groupe alcoxy en $C_1$-$C_4$, n, $R_1$ à $R_6$ et X ayant les significations indiquées dans la revendication 1.

7. Ethers d'oximes de formule (I) selon la revendication 1, dans lesquels A et B représentent chacun le soufre, Y un groupe alcoxy en $C_1$-$C_4$, n, $R_1$ à $R_6$ et X ayant les significations indiquées dans la revendication 1.

8. Ethers d'oximes de formule (I) selon la revendication 1, dans lesquels A représente l'oxygène, B le groupe méthine et Y l'hydrogène, n, $R_1$ à $R_6$ et X ayant les significations indiquées dans la revendication 1.

9. Ethers d'oximes de formule (I) selon la revendication 1, dans lesquels n est égal à 1, $R_1$, $R_2$ et $R_4$ représentent l'hydrogène, $R_3$ est en position 4 et représente l'hydrogène, un halogène, un groupe méthyle, difluorométhyle, trifluorométhyle, chlorodifluorométhyle, méthoxy, difluorométhoxy, trifluorométhoxy, chlorodifluorométhoxy, tétrafluoréthyle, tétrafluoréthoxy ou trifluorométhylsulfonyle, X représente un groupe alkyle en $C_1$-$C_3$ perfluoré ou un groupe chlorodifluorométhyle, A, B, $R_5$, $R_6$ et Y ayant les significations indiquées dans la revendication 1.

10. Ethers d'oximes de formule (I) selon la revendication 1, dans lesquels n est égal à 1, $R_1$, $R_2$ et $R_4$ représentent l'hydrogène, $R_3$ est en position 4 et représente l'hydrogène, le fluor, le chlore, un groupe méthyle, difluorométhyle, trifluorométhyle, chlorodifluorométhyle, méthoxy, difluorométhoxy, trifluorométhoxy, chlorodifluorométhoxy, tétrafluoréthyle, tétrafluoréthoxy ou trifluorométhylsulfonyle, X représente un groupe trifluorométhyle, chlorodifluorométhyle ou pentafluoréthyle et $R_5$ et $R_6$ forment ensemble un groupe 1,2-éthylène éventuellement substitué par 1 à 4 groupes méthyle, A, B et Y ayant les significations indiquées dans la revendication 1.

11. Ethers d'oximes de formule (I) selon la revendication 1, dans lesquels n est égal à 1, $R_1$, $R_2$ et $R_4$ représentent l'hydrogène, $R_3$ est en position 4 et représente l'hydrogène, le fluor, le chlore, un groupe trifluorométhyle ou trifluorométhoxy, X représente un groupe trifluorométhyle, chlorodifluorométhyle ou pentafluoréthyle et $R_5$ et $R_6$ forment ensemble un groupe 1,2-éthylène éventuellement substitué par 1 à 4 groupes méthyle.

12. Ethers d'oximes de formule (I) selon la revendication 1, dans lesquels n est égal à 1, $R_1$, $R_2$ et $R_4$ représentent l'hydrogène, $R_3$ est en position 2 et représente le fluor ou le chlore, X représente un groupe trifluorométhyle, chlorodifluorométhyle ou pentafluoréthyle et $R_5$ et $R_6$ forment ensemble un groupe 1,2-éthylène éventuellement substitué par 1 à 4 groupes méthyle.

13. Ethers d'oximes de formule (I) selon la revendication 1, dans lesquels n est égal à 1, $R_1$, $R_2$ et $R_4$ représentent l'hydrogène, $R_3$ est en position 3 et représente un groupe trifluorométhyle ou nitro, X représente un groupe trifluorométhyle, chlorodifluorométhyle ou pentafluoréthyle et $R_5$ et $R_6$ forment ensemble un groupe 1,2-éthylène éventuellement substitué par 1 à 4 groupes méthyle.

14. Le 1-phényl-1-(1,3-dioxolanne-2-ylméthoximino)-2,2,2-trifluoroéthane selon la revendication 1.

15. Le 1-(4-chlorophényl)-1-(1,3-dioxolanne-2-ylméthoximino)-2,2,2-trifluoréthane selon la revendication 1.

16. Le 1-(4-chlorophényl)-1-(2,2-diéthoxyéthoximino)-2,2,2-trifluoréthane selon la revendication 1.

17. Le 1-(4-chlorophényl)-1-(2,2-diméthoxyéthoximino)-2,2,2-trifluoréthane selon la revendication 1.

18. Le 1-(4-fluorophényl)-1-(1,3-dioxolanne-2-ylméthoximino)-2,2,2-trifluoréthane selon la revendication 1.

19. Le 1-(3,4-diméthylphényl)-1-(1,3-dioxolanne-2-ylméthoximino)-2,2,2-trifluoréthane selon la revendication 1.

20. Le 1-(fluorophényl)-1-(1,3-dioxolanne-4-méthyl-2-ylméthoximino)-2,2,2-trifluoréthane selon la revendication 1.

21. Le 1-phényl-1-(1,3-dioxanne-2-ylméthoximino)-2,2,2-trifluoréthane selon la revendication 1.

22. Le 1-(3-trifluorométhylnaphthyl)-1-(1,3-dioxanne-2-ylméthoximino)-2,2,2-trifluoréthane selon la revendication 1.

23. Le 1-phényl-(1,3-dioxolanne-2-ylméthoximino)-2,2,3,3,3-pentafluoréthane selon la revendication 1.

24. Le 1-(4-chlorophényl)-1-(1,3-dioxolanne-2-yléthyloximino)-2,2,2-trifluoréthane selon la revendication 1.

25. Le 1-(4-fluorophényl)-1-(1,3-dioxolanne-2-ylméthoximino)-2,2,2-trifluoréthane selon la revendication 1.

26. Le 1-(4-fluorophényl)-1-(1,3-dioxolanne-2-yléthylimino)-2,2,2-trifluoréthane selon la revendication 1.

27. Le 1-(4-chlorophényl)-1-(1,3-dioxolanne-4,5-diméthyl-2-ylméthoximino)-2,2,2-trifluoréthane selon la revendication 1.

28. Le 1-(3-trifluorométhylphényl)-1-(1,3-dioxolanne-4-éthyl-2-ylméthoximino)-2,2,2-trifluoréthane selon la revendication 1.

29. Procédé de préparation des éthers d'oximes de formule (I) selon la revendication 1, caractérisé en ce que l'on fait réagir un sel d'une oxime de formule (VI)

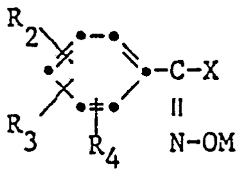 (VI)

dans laquelle M représente un cation de métal alcalin ou alcalino-terreux et $R_2$, $R_3$, $R_4$, X ont les significations indiquées dans la revendication 1, avec un halogénoacétal de formule (VII)

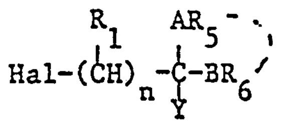 (VII)

dans laquelle Hal représente un atome d'halogène, en particulier le chlore ou le brome, et A, B, n, $R_1$, $R_5$, $R_6$ et Y ont les significations indiquées dans la revendication 1.

30. Procédé selon la revendication 29, caractérisé en ce que l'on utilise en tant que sel d'une oxime de formule (II) le sel de sodium ou de potassium.

31. Procédé de préparation des oximes de formule (I) selon la revendication 1, dans lesquelles A et B représentent chacun l'oxygène ou le soufre et Y représente un groupe alkyle en $C_1$-$C_4$ ou phényle, n, $R_1$ à $R_6$ et X ayant les significations indiquées dans la revendication 1, caractérisé en ce que l'on fait réagir dans un solvant ou diluant inerte un éther d'oxime de formule (X)

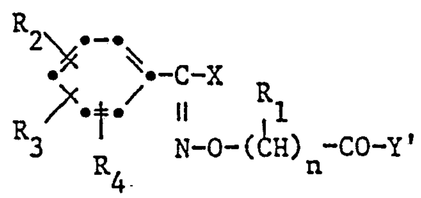 (X)

dans laquelle Y' représente un groupe alkyle en $C_1$-$C_4$ ou phényle, et n, $R_1$ à $R_4$ et X ont les significations indiquées dans la revendication 1, avec un orthoester formique de formule (XI)

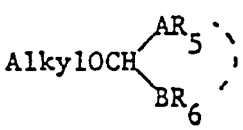 (XI)

dans laquelle Alkyl représente un groupe alkyle en $C_1$-$C_6$ et A, B, $R_5$ et $R_6$ ont les significations indiquées dans la revendication 1.

32. Procédé de préparation des composés de formule (I) selon la revendication 1, dans lesquels A et B représentent chacun l'oxygène ou le soufre, $R_5$ et $R_6$ forment ensemble un pont alkylène ou alcénylène contenant 2 à 6 chaînons et qui peut être substitué par des groupes alkyle en $C_1$-$C_4$, halogénoalkyle en $C_1$-$C_4$, alcoxy en $C_1$-$C_4$, alcényloxy en $C_2$-$C_4$, alcoxyalkyle en $C_2$-$C_8$ ou alcényloxyalkyle en $C_3$-$C_8$, et Y représente un groupe alcoxy en $C_1$-$C_4$, n, $R_1$ à $R_4$ et X ayant les significations indiquées dans la revendication 1, caractérisé en ce que l'on fait réagir un éther d'oxime de formule (XII)

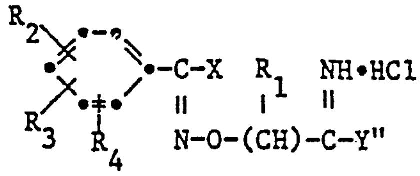 (XII)

dans laquelle Y'' représente un groupe alcoxy en $C_1$-$C_4$, n, R à $R_4$ et X ayant les significations indiquées dans la revendication 1, dans un solvant ou diluant inerte, avec un diol ou dithiol de formule (XIII)

$$HA-R_5-R_6-BH \qquad (XIII)$$

dans laquelle A et B représentent chacun l'oxygène ou le soufre et $R_5$ et $R_6$ forment un pont alkylène ou alcénylène contenant 2 à 6 chaînons et qui peut être substitué 1 à 4 fois par des groupes alkyle en $C_1$-$C_4$, halogénoalkyle en $C_1$-$C_4$, alcoxy en $C_1$-$C_4$, alcényloxy en $C_2$-$C_4$, alcoxyalcoxy en $C_2$-$C_8$ ou alcényloxyalkyle en $C_3$-$C_8$.

33. Procédé selon les revendications 29 à 32, caractérisé en ce que l'on effectue les réactions dans l'acétonitrile, le diméthylformamide ou le diméthylsulfoxyde servant de solvant ou de diluant.

34. Produit pour protéger les végétaux cultivés contre les dommages provoqués par les herbicides, caractérisé en ce qu'il contient, avec des véhicules et additifs inertes, un éther d'oxime de formule (I) selon la revendication 1 en tant que composant actif.

35. Produit selon la revendication 34, caractérisé en ce qu'il contient

a) un halogénoacétanilide ayant une activité herbicide ou un thiolcarbamate ayant une activité herbicide, et

b) un éther d'oxime de formule (I) selon la revendication 1 en tant qu'antidote.

36. Produit selon la revendication 35, caractérisé en ce qu'il contient

a) un halogénoacétanilide de formule (XIV)

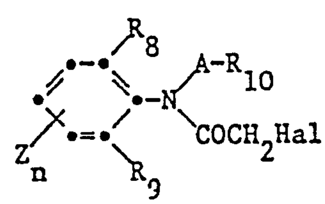 (XIV)

dans laquelle Hal représente un halogène, en particulier le chlore ou le brome, $R_8$ et $R_9$ représentent chacun, indépendamment l'un de l'autre, l'hydrogène, un halogène, un groupe alkyle inférieur, alcoxy, alkylthio, halogénoalkyle, alcoxyalkyle ou alkylthioalkyle, Z représente l'hydrogène, un halogène, un groupe alkyle inférieur, alcoxy, alkylthio,

halogénoalkyle, alcoxyalkyle ou alkylthioalkyle, les restes Z étant de préférence en position 3 par rapport à l'atome d'azote, n a une valeur de 0 à 3, A représente un groupe alkylène, en particulier méthylène, 1,1- et 1,2-éthylène, le groupe 1,2-éthylène pouvant être substitué par 1 ou 2 groupes alkyle inférieurs, et $R_{10}$ représente un groupe alcoxy inférieur, hydroxycarbonyle, alcoxycarbonyle, carbamoyle, N-alkylcarbamoyle, N,N-dialkylcarbamoyle, cyano, un reste hétérocyclique azoté éventuellement substitué, un groupe alcanoyle, un groupe benzyle éventuellement substitué, un groupe 1,3,4-oxadiazole-2-yle, 1,3,4-thiadiazole-2-yle, 1,3,4-triazole-3-yle ou 1,3,4-triazole-1-yle éventuellement substitué, et

b) un éther d'oxime de formule (I) selon la revendication 1 en tant qu'antidote.

37. Produit selon la revendication 35, caractérisé en ce qu'il contient

a) un thiolcarbamate de formules (XV) et (XVI)

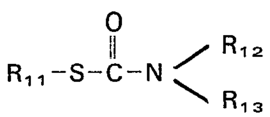

(XV)

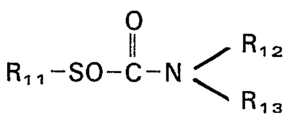

(XVI)

dans lesquelles $R_{11}$ représente un groupe alkyle inférieur, alcényle, chlorallyle, dichlorallyle, trichlorallyle, benzyle ou 4-chlorobenzyle, $R_{12}$ représente un groupe alkyle en $C_2$-$C_4$ et $R_{13}$ un groupe alkyle en $C_2$-$C_4$ ou cyclohexyle, les restes $R_{12}$ et $R_{13}$ pouvant former ensemble et avec l'atome d'azote auquel ils sont reliés un cycle hexahydro-1H-azépine, décahydroquinoléine ou 2-méthyldécahydroquinoléine, et

b) un éther d'oxime de formule (I) selon la revendication 1 en tant qu'antidote.

38. Utilisation des éthers d'oximes de formule (I) selon la revendication 1 pour la protection des végétaux cultivés contre les effets nocifs des herbicides.

39. Utilisation selon la revendication 36 des éthers d'oximes de formule (I) selon la revendication 1 pour la protection des cultures de céréales, de maïs et de sorgho contre les dommages provoqués par les halogénoacétanilides de formule (XIV) et les thiolcarbamates de formules (XV) et (XVI) tels que définis ci-dessus.

40. Procédé pour protéger les végétaux cultivés contre les dommages provoqués par l'application d'herbicides, caractérisé en ce que l'on traite par une quantité efficace d'un éther d'oxime de formule (I) selon la revendication 1

a) l'aire de culture pour le végétal avant ou durant l'application de l'herbicide, ou

b) les graines ou les plants de la plante ou la plante elle-même.